Europäisches Patentamt

European Patent Office

Office européen des brevets

(19)

(11) EP 0 728 205 B1

(12) EUROPEAN PATENT SPECIFICATION

(45) Date of publication and mention
of the grant of the patent:
**16.10.2002 Bulletin 2002/42**

(51) Int Cl.[7]: **C12N 15/52**, C07H 21/02,
C12N 9/00, A61K 31/70

(21) Application number: **95901907.6**

(22) Date of filing: **10.11.1994**

(86) International application number:
**PCT/US94/13129**

(87) International publication number:
**WO 95/013380 (18.05.1995 Gazette 1995/21)**

(54) **REAGENT FOR TREATMENT OF ARTHRITIC CONDITIONS**

REAGENZ ZUR BEHANDLUNG VON ARTHRITISCHEN ZUSTAENDEN

REACTIF POUR LE TRAITEMENT D'ETATS ARTHRITIQUES

(84) Designated Contracting States:
**AT BE CH DE DK ES FR GB GR IE IT LI LU MC NL
PT SE**

(30) Priority: **12.11.1993 US 152487**

(43) Date of publication of application:
**28.08.1996 Bulletin 1996/35**

(73) Proprietor: **RIBOZYME PHARMACEUTICALS,
INC.
Boulder, CO 80301 (US)**

(72) Inventors:
• **DRAPER, Kenneth, G.
Boulder, CO 80301 (US)**
• **PAVCO, Pamela
LaFayette, CO 80026 (US)**
• **McSWIGGEN, James
Boulder, CO 80301 (US)**

• **GUSTOFSON, John
Boulder, CO 80301 (US)**

(74) Representative: **Nobbe, Matthias, Dr. et al
Viering, Jentschura & Partner,
Essener Strasse 5
46047 Oberhausen (DE)**

(56) References cited:
**WO-A-90/13300            WO-A-94/02595**

• **PROCEEDINGS OF THE AMERICAN
ASSOCIATION FOR CANCER RESEARCH, vol.
32, March 1991 page 277 BORCHERS, A. &
BOWDEN, G. 'Generation of invasive mouse
keratinocyte cell lines down-regulated for
stromelysin protein expression by transfection
with antisense RNA' & 82ND ANNUAL MEETING
OF THE AACR; HOUSTON TEXAS, 15 May 1991
- 18 May 1991**

EP 0 728 205 B1

**Description**

Background of the Invention

This application is a continuation-in-part of Draper, entitled "Method and Reagent For Treatment of Arthritic Conditions", filed December 7, 1992, the whole of which, including drawings, is hereby incorporated by reference herein.

This invention relates to methods for inhibition of osteoarthritis, in particular, inhibition of genetic expression which leads to a reduction or elimination of extracellular matrix digestion by matrix metalloproteinases.

There are several types of arthritis, with osteoarthritis and rheumatoid arthritis being predominant. Osteoarthritis is a slowly progressive disease characterized by degeneration of articular cartilage with proliferation and remodeling of subchondral bone. It presents with a clinical picture of pain, deformity, and loss of joint motion. Rheumatoid arthritis is a chronic systemic inflammatory disease. Rheumatoid arthritis may be mild and relapsing or severe and progressive, leading to joint deformity and incapacitation.

Arthritis is the major contributor to functional impairment among the older population. It is the major cause of disability and accounts for a large proportion of the hospitalizations and health care expenditures of the elderly. Arthritis is estimated to be the principal cause of total incapacitation for about one million persons aged 55 and older and is thought to be an important contributing cause for about one million more.

Estimating the incidence of osteoarthritis is difficult for several reasons. First, osteoarthritis is diagnosed objectively on the basis of reading radiographs, but many people with radiologic evidence of disease have no obvious symptoms. Second, the estimates of prevalence are based upon clinical evaluations because radiographic data is not available for all afflicted joints. In the NHANESI survey of 1989, data were based upon a thorough musculoskeletal evaluation during which any abnormalities of the spine, knee, hips, and peripheral joints were noted as well as other specific diagnoses. Based on these observations, 12% of the US population between 25 and 74 years of age have osteoarthritis.

It is generally agreed that rheumatoid arthritis has a world-wide distribution and affects all racial and ethnic groups. The exact prevalence in the US is unknown but has been estimated to range between 0.5% and 1.5%. Rheumatoid arthritis occurs at all age levels and generally increases in prevalence with advancing age. It is 2-3 times more prevalent in women than in men and peak incidence occurs between 40-60 years of age. In addition to immunological factors, environmental, occupational and psychosocial factors have been studied for potential etiologic roles in the disease.

The extracellular matrix of multicellular organisms plays an important role in the formation and maintenance of tissues. The meshwork of the extracellular matrix is deposited by resident cells and provides a framework for cell adhesion and migration, as well as a permeability barrier in cell-cell communication. Connective tissue turnover during normal growth and development or under pathological conditions is thought to be mediated by a family of neutral metalloproteinases, which are zinc-containing enzymes that require calcium for full activity. The regulation of metalloproteinase expression is cell-type specific and may vary among species.

The best characterized of the matrix metalloproteinases, interstitial collagenase (MMP-1), is specific for collagen types I, II, and III. MMP-1 cleaves all three α chains of the triple helix at a single point initiating sequential breakdown of the interstitial collagens. Interstitial collagenase activity has been observed in rheumatoid synovial cells as well as in the synovial fluid of patients with inflammatory arthritis. Gelatinases (MMP-2) represent a subgroup of the metalloproteinases consisting of two distinct gene products; a 70 kDa gelatinase expressed by most connective tissue cells, and a 92 kDa gelatinase expressed by inflammatory phagocytes and tumor cells. The larger enzyme is expressed by macrophages, SV-40 transformed fibroblasts, and neutrophils. The smaller enzyme is secreted by H-ras transformed bronchial epithelial cells and tumor cells, as well as normal human skin fibroblasts. These enzymes degrade gelatin (denatured collagen) as well as native collagen type XI. Stromelysin (MMP-3) has a wide spectrum of action on molecules composing the extracellular matrix. It digests proteoglycans, fibronectin, laminin, type IV and IX collagens and gelatin, and can remove the N-terminal propeptide region from procollagen, thus activating the collagenase. It has been found in human cartilage extracts, rheumatoid synovial cells, and in the synovium and chondrocytes of joints in rats with collagen-induced arthritis.

Both osteoarthritis and rheumatoid arthritis are treated mainly with compounds that inhibit cytokine or growth-factor induced synthesis of the matrix metalloproteinases which are involved in the extracellular matrix destruction observed in these diseases. Current clinical treatments rely upon dexamethasone and retinoid compounds, which are potent suppressors of a variety of metalloproteinases. The global effects of dexamethasone and retinoid treatment upon gene expression in treated cells make the development of alternative therapies desirable, especially for long term treatments. Recently, it was shown that gamma-interferon suppressed lipopolysaccharide induced collagenase and stromelysin production in cultured macrophages. Also, tissue growth factor-β (TGF-β) has been shown to block epidermal growth factor (EGF) induction of stromelysin synthesis in vitro. Experimental protocols involving gene therapy approaches include the controlled expression of the metalloproteinase inhibitors TIMP-1 and TIMP-2. Of the latter three approaches, only gamma-interferon treatment is currently feasible in a clinical application.

Summary of the Invention

Applicant notes that the inhibition of collagenase and stromelysin production in the synovial membrane of joints can be accomplished using ribozymes and antisense molecules. Ribozyme treatment can be a partner to current treatments which primarily target immune cells reacting to pre-existing tissue damage. Early ribozyme or antisense treatment which reduces the collagenase or stromelysin-induced damage can be followed by treatment with the antiinflammatories or retinoids, if necessary. In this manner, expression of the proteinases can be controlled at both transcriptional and translational levels. Ribozyme or antisense treatment can be given to patients expressing radiological signs of osteoarthritis prior to the expression of clinical symptoms. Ribozyme or antisense treatment can impact the expression of stromelysin without introducing the non-specific effects upon gene expression which accompany treatment with the retinoids and dexamethasone. The ability of stromelysin to activate procollagenase indicates that a ribozyme or antisense molecule which reduces stromelysin expression can also be used in the treatment of both osteoarthritis (which is primarily a stromelysin-associated pathology) and rheumatoid arthritis (which is primarily related to enhanced collagenase activity).

While a number of cytokines and growth factors induce metalloproteinase activities during wound healing and tissue injury of a pre-osteoarthritic condition, these molecules are not preferred targets for therapeutic intervention. Primary emphasis is placed upon inhibiting the molecules which are responsible for the disruption of the extracellular matrix, because most people will be presenting radiologic or clinical symptoms prior to treatment. The most versatile of the metalloproteinases (the molecule which can do the most structural damage to the extracellular matrix, if not regulated) is stromelysin. Additionally, this molecule can activate procollagenase, which in turn causes further damage to the collagen backbone of the extracellular matrix. Under normal conditions, the conversion of prostromelysin to active stromelysin is regulated by the presence of inhibitors called TIMPs (tissue inhibitors of MMP). Because the level of TIMP in synovial cells exceeds the level of prostromelysin and stromelysin activity is generally absent from the synovial fluid associated with non-arthritic tissues, the toxic effects of inhibiting stromelysin activity in non-target cells should be negligible.

Thus, the invention features use of ribozymes or antisense molecules to treat or prevent arthritis, particularly osteoarthritis, e.g., by inhibiting the synthesis of the prostromelysin molecule in synovial cells, or by inhibition of other matrix metalloproteinases discussed above. Cleavage of targeted mRNAs (stromelysin mRNAs, including stromelysin 1, 2, and 3, and collagenase) expressed in macrophages, neutrophils and synovial cells represses the synthesis of the zymogen form of stromelysin, prostromelysin. Those in the art will recognize the other potential targets discussed above are also suitable for treatment with ribozymes, which will reduce the risk or occurrence of pathologic degradation of the extracellular matrix such as the collagenase and gelatinase metalloproteinases, other proteinases which can activate the proenzyme forms of the metalloproteinases in synovial fluid or cartilaginous cells, cytokines or growth factors which activate the expression of the metalloproteinases and adhesion molecules which attract macrophage and neutrophils to the areas of tissue injury.

Ribozymes are RNA molecules having an enzymatic activity which is able to repeatedly cleave other separate RNA molecules in a nucleotide base sequence specific manner. It is said that such enzymatic RNA molecules can be targeted to virtually any RNA transcript and efficient cleavage has been achieved _in vitro_. Kim et al., 84 Proc. Nat. Acad. of Sci. USA 8788, 1987; Haseloff and Gerlach, 334 Nature 585, 1988; Cech, 260 JAMA 3030, 1988; and Jefferies et al., 17 Nucleic Acid Research 1371, 1989.

Ribozymes act by first binding to a target RNA. Such binding occurs through the target RNA binding portion of a ribozyme which is held in close proximity to an enzymatic portion of the RNA which acts to cleave the target RNA. Thus, the ribozyme first recognizes and then binds a target RNA through complementary base-pairing, and once bound to the correct site, acts enzymatically to cut the target RNA. Strategic cleavage of such a target RNA will destroy its ability to direct synthesis of an encoded protein. After a ribozyme has bound and cleaved its RNA target it is released from that RNA to search for another target and can repeatedly bind and cleave new targets.

The enzymatic nature of a ribozyme is advantageous over other technologies, such as antisense technology (where a nucleic acid molecule simply binds to a nucleic acid target to block its translation) since the effective concentration of ribozyme necessary to effect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding, but also on the mechanism by which the molecule inhibits the expression of the RNA to which it binds. That is, the inhibition is caused by cleavage of the RNA target and so specificity is defined as the ratio of the rate of cleavage of the targeted RNA over the rate of cleavage of non-targeted RNA. This cleavage mechanism is dependent upon factors additional to those involved in base pairing. Thus, it is thought that the specificity of action of a ribozyme is greater than that of antisense oligonucleotide binding the same RNA site.

This class of chemicals exhibits a high degree of specificity for cleavage of the intended target mRNA. Consequently, the ribozyme agent will only affect cells expressing that particular gene, and will not be toxic to normal tissues.

The invention can be used to treat or prevent (prophylactically) osteoarthritis or other pathological conditions which are mediated by metalloproteinase activation. The preferred administration protocol is *in vivo* administration to reduce the level of stromelysin activity.

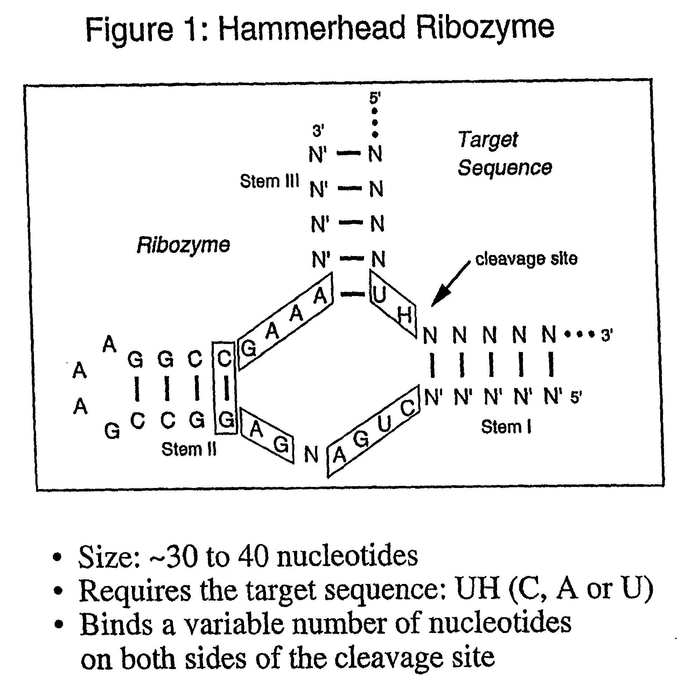

Thus, in the first aspect, the invention features an enzymatic RNA molecule (or ribozyme) which cleaves mRNA associated with development or maintenance of an arthritic condition, e.g., mRNA encoding stromelysin, and in particular, those mRNA targets disclosed in the tables below, which include both hammerhead and hairpin target sites. In each case the site is flanked by regions to which appropriate substrate binding arms can be synthesized and an appropriate hammerhead or hairpin motif can be added to provide enzymatic activity, by methods described herein and known in the art. For example, referring to Figure 1, arms I and III are modified to be specific substrate-binding arms, and arm II remains essentially as shown.

By "enzymatic RNA molecule" it is meant an RNA molecule which has complementarity in a substrate binding region to a specified mRNA target, and also has an enzymatic activity which is active to specifically cleave that mRNA. That is, the enzymatic RNA molecule is able to intermolecularly cleave mRNA and thereby inactivate a target mRNA molecule. This complementarity functions to allow sufficient hybridization of the enzymatic RNA molecule to the target RNA to allow the cleavage to occur. One hundred percent complementarity is preferred, but complementarity as low as 50-75% may also be useful in this invention. For *in vivo* treatment, complementarity between 30 and 45 bases is preferred; although lower numbers are also useful.

In preferred embodiments, the enzymatic RNA molecule is formed in a hammerhead motif, but may also be formed in the motif of a hairpin, hepatitis delta virus, group I intron or RNAseP-like RNA (in association with an RNA guide sequence). Examples of such hammerhead motifs are described by Rossi et al., 8 Aids Research and Human Retroviruses 183, 1992, of hairpin motifs by Hampel et al., "RNA Catalyst for Cleaving Specific RNA Sequences", filed September 20, 1989, which is a continuation-in-part of U.S. Serial No. 07/247,100, filed September 20, 1988; Hampel and Tritz, 28 Biochemistry 4929, 1989; and Hampel et al., 18 Nucleic Acids Research 299, 1990, and an example of the hepatitis delta virus motif is described by Perrotta and Been, 31 Biochemistry 16, 1992, of the RNAseP motif by Guerrier-Takada, et al., 35 Cell 849, 1983, and of the group I intron by Cech et al., U.S. Patent 4,987,071. All the publications are hereby incorporated by reference herein. These specific motifs are not limiting in the invention and those skilled in the art will recognize that all that is important in an enzymatic RNA molecule of this invention is that it has a specific substrate binding site which is complementary to one or more of the target gene RNA regions, and that it have nucleotide sequences within or surrounding that substrate binding site which impart an RNA cleaving activity to the molecule.

In a second related aspect, the invention features a mammalian cell which includes an enzymatic RNA molecule as described above. Preferably, the mammalian cell is a human cell.

In a third related aspect, the invention features an expression vector which includes nucleic acid encoding an enzymatic RNA molecule described above, located in the vector, e.g., in a manner which allows expression of that enzymatic RNA molecule within a mammalian cell.

In a fourth related aspect, the invention features a method for treatment of an arthritic condition by administering to a patient an enzymatic RNA molecule as described above.

The invention provides a class of chemical cleaving agents which exhibit a high degree of specificity for the mRNA causative of an arthritic condition. Such enzymatic RNA molecules can be delivered exogenously or endogenously to infected cells. In the preferred hammerhead motif the small size (less than 40 nucleotides, preferably between 32 and 36 nucleotides in length) of the molecule allows the cost of treatment to be reduced.

One ribozyme delivered for treatment reported to date (by Rossi et al., 1992, supra) is an *in vitro* transcript having a length of 142 nucleotides. Synthesis of ribozymes greater than 100 nucleotides in length is very difficult using automated methods, and the therapeutic cost of such molecules is prohibitive. Delivery of ribozymes by expression vectors is primarily feasible using only *ex vivo* treatments. This limits the utility of this approach. In this invention, an alternative approach uses smaller ribozyme motifs (e.g., of the hammerhead structure, shown generally in Fig. 1) and exogenous delivery. The simple structure of these molecules also increases the ability of the ribozyme to invade targeted regions of the mRNA structure. Thus, unlike the situation when the hammerhead structure is included within longer transcripts, there are no non-ribozyme flanking sequences to interfere with correct folding of the ribozyme structure, as well as complementary binding of the ribozyme to the mRNA target.

The enzymatic RNA molecules of this invention can be used to treat arthritic or prearthritic conditions. Such treatment can also be extended to other related genes in nonhuman primates. Affected animals can be treated at the time of arthritic risk detection, or in a prophylactic manner. This timing of treatment will reduce the chance of further arthritic damage.

Ribozymes of this invention may be used as diagnostic tools to examine genetic drift and mutations within diseased cells. The close relationship between ribozyme activity and the structure of the target RNA allows the detection of mutations in any region of the molecule which alters the base-pairing and three-dimensional structure of the target RNA. By using multiple ribozymes described in this invention, one may map nucleotide changes which are important to RNA structure and function *in vitro,* as well as in cells and tissues. Cleavage of target RNAs with ribozymes may be used to inhibit gene expression and define the role (essentially) of specified gene products in the progression of disease. In this manner, other genetic targets may be defined as important mediators of the disease. These experiments will lead to better treatment of the disease progression by affording the possibility of combinational therapies (e.g., multiple ribozymes targeted to different genes, ribozymes coupled with known small molecule inhibitors, or intermittent treatment with combinations of ribozymes and/or other chemical or biological molecules). Other in vitro uses of ribozymes and antisense molecules of this invention are well known in the art, and include detection of the presence of mRNA associated with an arthritic condition. Such RNA is detected by determining the presence of a cleavage product after treatment with a ribozyme using standard methodology.

Other features and advantages of the invention will be apparent from the following description of the preferred embodiments thereof, and from the claims.

## Description of the Preferred Embodiments

The drawing will first briefly be described.

## Drawing

Fig. 1 is a diagrammatic representation of a hammerhead motif ribozyme showing stems I, II and III (marked (I), (II) and (III) respectively) interacting with a target region. The 5' and 3' ends of both ribozyme and target are shown. Dashes indicate base-paired nucleotides.

## Target Sites

Two types of computational technologies are available for aiding in the identification of target sites and design of active ribozymes. First, DNA/RNA sequence analysis software is employed to identify sequence motifs necessary for ribozyme cleavage and to look for sequence conservation between different sources of the target organism so that ribozymes with the broadest possible target range can be designed. Second, RNA folding algorithms are employed to predict the secondary structure of both ribozyme and target RNA in an attempt to identify combinations of ribozyme and target site that will successfully associate prior to ribozyme cleavage. The RNA folding algorithms utilize a set of thermodynamic parameters obtained from measurements on short RNA duplexes; while these rules give reasonable predictions of secondary structure for a small set of highly structured RNAs, they reamin largely untested for predicting the structure of messenger RNAs.

Currently, five different classes of ribozymes have been discovered. The largest of these ribozymes (group I introns and RNAse P RNA) contain from 200 to over 1000 nucleotides of sequence (70kD to over 350 kD) and fold into complex secondary and tertiary structures. The smaller ribozyme motifs (hammerhead, hairpin, and HDV) consist of 60 nucleotides or less (<21 kD) and consequently fold into less complex structures. This article will focus on targeting of the hammerhead ribozyme (Fig. 1) because of its small size and well-defined structure. The general strategy described here should be applicable to targeting by other ribozymes as well.

The hammerhead ribozyme motif is shown in Fig. 1. The ribozyme and substrate complex consists of three base-paired helices surrounding a central core region. Most of the nucleotides within the central core are fixed (boxed sequences); replacement of these nucleotides results in dramatic reductions in ribozyme cleavage activity (Ruffner et al., 29 Biochem 10695, 1990). With the exception of the two base-pairs closing stems II and III, the sequences within the three stem regions are arbitrary; the only requirement is that the stems be allowed to form. Figure 1 shows stem II formed from intramolecular base-pairing interactions to form a hairpin loop; the other two stems are made by base-pairing between two different RNAs. Any, or all, of the three stem regions can be formed from intramolecular or intermolecular base-pairing interactions. If two of the stems are linked to themselves, the ribozyme cleavage reaction becomes an intramolecular (cis-cleavage) reaction. The arrangement shown in Figure 1 yields a trans-cleaving (intermolecular) ribozyme with most of the fixed (boxed) sequences located in the ribozyme portion of the structure. Thus, the target RNA only requires a primary sequence consisting of a uridine nucleotide (U) followed by any base except guanosine--where H represents adenosine (A), cytidine (C), or uridine (U). In an mRNA molecule containing a random distribution of all 4 bases, the UH sequence would be expected to occur once every 5-6 nucleotides on average.

In addition to the UH primary sequence, the target site must have flanking sequences that promote binding of an appropriately designed hammerhead ribozyme. Successful association of a ribozyme with its cognate target site primarily will depend on three things (I) the binding free energy of ribozyme to target site, (ii) the propensity of ribozyme to fold up into competing self-structure, and (iii) the propensity of the target RNA to fold up into competing self-structure (additionally, RNA binding proteins are likely to affect the binding of ribozymes inside the cell). The binding free energy of ribozyme to target site can be increased by increasing the length of the target-binding regions (stems I and III in Figure 1), however that will also increase the potential for forming competing self-structures.

As depicted in Figure 1, the hammerhead ribozyme, alone, should fold into a single hairpin loop with two single-stranded arms ready to bind the target sequence. There are a number of ways to reduce or avoid the potential for unwanted alternative folds in ribozymes. One way to avoid unwanted alternative folds is simply to look at ribozymes targeted to different sites on the same target RNA molecule. Most target RNAs have many potential ribozyme cleavage sites (sequences containing the required UH sequence). To some extent, ribozymes that don't fold correctly simply can be discarded in favor of sites where targeted ribozymes are predicted to fold correctly.

For a given ribozyme, reduction or elimination of unwanted structures can be achieved through redesign of the non-conserved parts of the ribozyme. As noted earlier, the unboxed nucleotides in Figure 1 can generally be altered without affecting ribozyme activity. All three stems can be changed as long as stem formation is maintained; in addition, a single nucleotide within the central core can be changed. Altering the sequence of stem II or the length of the substrate binding arms (stems I and III in Figure 1) are two primary means of changing the predicted structure of the ribozyme.

Changing the length of the substrate binding arms will also change the binding free energy between ribozyme and target. Thus, prediction of the most stable structure between target and ribozyme should also be examined. No known folding algorithm allows two unattached RNA molecules to be folded together and be analyzed in that way; if such an algorithm was available the structure analysis would depend on the concentration of the two RNAs in addition to their sequence. An interim solution to this problem is to artificially attach the ribozyme to a complementary "target" sequence and perform a folding analysis on the combined sequence.

Structure prediction becomes more difficult and less certain as the size of the RNA molecule increases. There are a number of reasons for this. First, the estimates of nearest-neighbor base-pairing free energies have an uncertainty of 5-10% in simple oligonucleotide duplexes, and those uncertainties are compounded with increasing numbers of base-pairs. Second, the free energies of more complex structures--such as multi-branched loops--are not well defined experimentally and are likely not to conform strictly to the additivity assumptions of the nearest-neighbor model. Third, most parts of messenger RNAs may not be evolutionarily selected for one unique structure; a whole set of alternative structures may be in equilibrium with each other. Thus, current free energy minimization algorithms provide a suitable starting point for experimentally determining the structure of some RNA molecules (rRNA, tRNA, group I introns, etc.), while a given mRNA is likely to exist as a population of molecules having different structures with similar free energies. Finally, long RNAs may fold into locally stable structures that are kinetically blocked from reaching the global free energy minimum; if so, structure predictions based on global free energy minima will miss the biologically relevant structure.

Of interest in the prediction of accessible sites is not the identification of a single folded structure for a given target mRNA. Instead, the objective of this exercise is to assess the likelihood of unpaired (or substantially unpaired) sites that could be a ribozyme target within an RNA molecule, rather than identifying specific base-pairing interactions between two sites or regions. Unstructured sites are likely easier to predict since there is a single way to have a base unpaired, but a myriad of ways to pair that base in a folded structure. Ambiguities in thermodynamic parameters--and the possibility that each mRNA exists as a population of different folded structures--suggests that a stochastic approach to the evaluation of accessible sites may be appropriate. AS a starting point, two measures of mRNA accessibility are being examined and compared to experimentally determined values.

Global accessibility calculates the average accessibility of target sites based on overall minimum free-energy folding. The entire RNA molecule is folded at one time, and a set of 100-200 optimal and sub-optimal structures are generated within 5-10% of the minimal free energy. Similar structure are excluded from the assemblage so that a certain number of structural differences exist between the alternative folded structures (see the description of the distance parameter in Zuker, 244 Science 48, 1989). For each structure, the number of unpaired bases is tabulated for a region around each potential ribozyme (UH) cleavage site within the sequence. The size of the region around each site is determined by the ribozyme being designed to that site; a 15 nucleotide site size has been chosen for these initial studies. Global accessibility is calculated by tabulating the number of unpaired nucleotides in a given (15 nucleotide) target sequence and then determining the average number of unpaired nucleotides over the length of the target site and over the population of folded structures. A high score (up to 100%) represents a high expectation that the bases within a given site are unpaired at any given time. The calculated average accessibility is relatively independent of the degree of structural differences between the alternative folded structures (i.e., changes in the distance parameter) and the number of sub-optimal structures analyzed (data not shown).

Local stability calculates the strength of local folded structures to model possible local trapping of sequences. Overlapping RNa segments (30-50 nucleotides) are folded by energy minimization, and the minimum free energy of each segment is tabulated. For an RNA of length, N, there will be (N-n+1) RNA segments of length, n. The relative local stability of each segment is calculated by comparing its energy with the average energy of all other folded segments of the RNA (i.e., stability score = $(E_i-E_{ab})\sigma_{db}$; where $E_i$ is energy of the segment i, $E_{ab}$=average energy of all segments in the sequence, and $\sigma_{db}$ is the standard deviation for the free energy distribution. If $E_i$ is larger than $E_{ab}$ the score is positive and segment i is considered to be unstable and more easily melted out relative to the average folded segment. If the target RNA is predicted to fold into a very stable structure, regions with high stability scores may still be relatively difficult to melt open. Thus, the actual energy per segment should be examined in addition to the stability score. RNA segment size (30-50 nucleotides) is chosen to accommodate relatively large local structures (e.g., 13-23 base-pair stem loops) while keeping the segments small enough to actually reflect structures around the target sites.

Ribozymes targeting selected regions of mRNA associated with arthritic disease are chosen to cleave the target RNA in a manner which preferably inhibits translation of the RNA. Genes are selected such that inhibition of translation will preferably inhibit cell replication, e.g., by inhibiting production of a necessary protein or prevent production of an undesired protein, e.g., stromelysin. Selection of effective target sites within these critical regions of mRNA entails testing the accessibility of the target RNA to hybridization with various oligonucleotide probes. These studies can be performed using RNA or DNA probes and assaying accessibility by cleaving the hybrid molecule with RNAseH (see below and McSwiggen, Assay for Ribozyme Target Site Accessibility, U.S.S.N. 07/884,073, filed May 14, 1992, hereby incorporated by reference herein). Alternatively, such a study can use ribozyme probes designed from secondary structure predictions of the mRNAs, and assaying cleavage products by polyacrylamide gel electrophoresis (PAGE), to detect the presence of cleaved and uncleaved molecules.

The following is but one example of a method by which suitable target sites can be identified and is not limiting in this invention. Generally, the method involves identifying potential cleavage sites for a hammerhead ribozyme, and then testing each of these sites to determine their suitability as targets by ensuring that secondary structure formation is minimal.

The mRNA sequences are compared in an appropriate target region. Putative ribozyme cleavage sites are found. These sites represent the preferable sites for hammerhead ribozyme cleavage within these target mRNAs. Table 1 shows an example of such sites for hammerhead ribozymes.

Table 1

----------------------------------------------------------------------

Nucleotide

| Number | Sequence | SEQ. ID. NO. |
|---|---|---|
| 20 | UAGAGCUAAGUAAAGCCAG | ID. NO. 01 |
| 126 | ACACCAGCAUGAA | ID. NO. 02 |
| 147 | AGAAAUAUCUAGA | ID. NO. 03 |

7

| 171 | ACCUCAAAAAAGAUGUGAAACAGU | ID. NO. 04 |
| 240 | AAAUGCAGAAGUUC | ID. NO. 05 |
| 287 | GACACUCUGGAGGUGAUGCGCAAGCCCAGGUGU | ID. NO. 06 |
| 327 | CUGAUGUUGGUCACUUCAGAAC | ID. NO. 07 |
| 357 | GCAUCCCGAAGUGGAGGAAAACCCACCUUACAU | ID. NO. 08 |
| 402 | AUUAUACACCAGAUUUGCCAAAAGAUG | ID. NO. 09 |
| 429 | CUGUUGAUUCUGCUGUUGAGA | ID. NO. 10 |
| 455 | CUGAAAGUCUGGGAAGAGGUGA | ID. NO. 11 |
| 513 | CUGAUAUAAUGA | ID. NO. 12 |
| 592 | UGCCUAUGCCCC | ID. NO. 13 |
| 624 | AUGCCCACUUUGAUGAUGAUGAACAAUGGACA | ID. NO. 14 |
| 679 | AUUUCUCGUUGCUGCUCAUG | ID. NO. 15 |
| 725 | CACUCAGCCAACACUGA | ID. NO. 16 |
| 801 | AAGAUGAUAUAAAUGGCAUUCAGUCC | ID. NO. 17 |
| 827 | CUCUAUGGACCUCCCCCUGACUCCCCU | ID. NO. 18 |
| 859 | CCCCCUGGUACCCA | ID. NO. 19 |
| 916 | UCCUGCUUUGUCCUUUGAUGCUGUCAGCAC | ID. NO. 20 |
| 958 | AAUCCUGAUCUUUAAAGA | ID. NO. 21 |
| 975 | CAGGCACUUUUGGCGCAAAUCCC | ID. NO. 22 |
| 1018 | AUUGCAUUUGAUCUCUUCAUUUUGGCCAUC | ID. NO. 23 |
| 1070 | GCAUAUGAAGUUA | ID. NO. 24 |
| 1203 | AAAUCGAUGCAGCCAUUUCUGA | ID. NO. 25 |
| 1274 | UUUGAUGAGAAGAGAAAUUCCAUGGAGC | ID. NO. 26 |
| 1302 | CAGGCUUUCCCAAGCAAAUAGCUGAAGAC | ID. NO. 27 |
| 1420 | CCCAAAUGCAAAG | ID. NO. 28 |
| 1485 | AUGUAGAAGGCACAAUAUGGGCACUUUAAA | ID. NO. 29 |
| 1623 | UCUUGCCGGUCAUUUUUAUGUUAU | ID. NO. 30 |
| 1665 | GCUGCUGCUUAGC | ID. NO. 31 |
| 1733 | CAACAGACAAGUGACUGUAUCU | ID. NO. 32 |
| 1769 | CUUAUUUAAUA | ID. NO. 33 |

--------------------------------------------------------------------

Short RNA substrates corresponding to each of the mRNA sites are designed. Each substrate is composed of two to three nucleotides at the 5' and 3' ends that will not base pair with a corresponding ribozyme recognition region. The unpaired regions flanked a central region of 12-14 nucleotides to which complementary arms in the ribozyme are designed.

The structure of each substrate sequence is predicted using a PC fold computer program. Sequences which give a positive free energy of binding are accepted. Sequences which give a negative free energy are modified by trimming one or two bases from each of the ends. If the modified sequences are still predicted to have a strong secondary structure, they are rejected.

After substrates are chosen, ribozymes are designed to each of the RNA substrates. Ribozyme folding is also

8

analyzed using PC fold or Mullfold, which programs are well known in the art.

Ribozyme molecules are sought which form hammerhead motif stem II (see Fig. 1) regions and contain flanking arms (stems I and III) which are devoid of intramolecular base pairing. Often the ribozymes are modified by trimming a base from the ends of the ribozyme, or by introducing additional base pairs in stem II to achieve the desired fold. Ribozymes with incorrect folding are rejected. After substrate/ribozyme pairs are found to contain correct intramolecular structures, the molecules are folded together to predict intermolecular interactions. A schematic representation of a ribozyme with its coordinate base pairing to its cognate target sequence is shown in Fig. 1.

Those targets thought to be useful as ribozyme targets can be tested to determine accessibility to nucleic acid probes in a ribonuclease H assay (see below). This assay provides a quick test of the use of the target site without requiring synthesis of a ribozyme. It can be used to screen for sites most suited for ribozyme attack.

Synthesis of Ribozymes

Ribozymes useful in this invention can be produced by gene transcription as described by Cech, supra, or by chemical synthesis. Chemical synthesis of RNA is similar to that for DNA synthesis. The additional 2'-OH group in RNA, however, requires a different protecting group strategy to deal with selective 3'-5' internucleotide bond formation, and with RNA susceptibility to degradation in the presence of bases. The recently developed method of RNA synthesis utilizing the t-butyldimethylsilyl group for the protection of the 2' hydroxyl is the most reliable method for synthesis of ribozymes. The method reproducibly yields RNA with the correct 3'-5' internucleotide linkages, with average coupling yields in excess of 99%, and requires only a two-step deprotection of the polymer.

A method, based upon H-phosphonate chemistry of phosphoroamidites gives a relatively lower coupling efficiency than a method based upon phosphoroamidite chemistry. This is a problem for synthesis of DNA as well. A promising approach to scale-up of automatic oligonucleotide synthesis has been described recently for the H-phosphonates. A combination of a proper coupling time and additional capping of "failure" sequences gave high yields in the synthesis of oligodeoxynucleotides in scales in the range of 14 μmoles with as little as 2 equivalents of a monomer in the coupling step. Another alternative approach is to use soluble polymeric supports (e.g., polyethylene glycols), instead of the conventional solid supports. This method can yield short oligonucleotides in hundred milligram quantities per batch utilizing about 3 equivalents of a monomer in a coupling step.

Various modifications to ribozyme structure can be made to enhance the utility of ribozymes. Such modifications will enhance shelf-life, half-life in vitro, stability, and ease of introduction of such ribozymes to the target site, e. g., to enhance penetration of cellular membranes, and confer the ability to recognize and bind to targeted cells.

Exogenous delivery of ribozymes benefits from chemical modification of the backbone, e.g., by the overall negative charge of the ribozyme molecule being reduced to facilitate diffusion across the cell membrane. The present strategies for reducing the oligonucleotide charge include: modification of internucleotide linkages by methylphosphonates, use of phosphoramidites, linking oligonucleotides to positively charged molecules, and creating complex packages composed of oligonucleotides, lipids and specific receptors or effectors for targeted cells. Examples of such modifications include sulfur-containing ribozymes containing phosphorothioates and phosphorodithioates as internucleotide linkages in RNA. Synthesis of such sulfur-modified ribozymes is achieved by use of the sulfur-transfer reagent, $^3$H-1,2-benzenedithiol-3-one 1,1-dioxide. Ribozymes may also contain ribose modified ribonucleotides. Pyrimidine analogues are prepared from uridine using a procedure employing diethylamino sulphur trifluoride (DAST) as a starting reagent. Ribozymes can also be either electrostatically or covalently attached to polymeric cations for the purpose of reducing charge. The polymer can be attached to the ribozyme by simply converting the 3'-end to a ribonucleoside dialdehyde which is obtained by a periodate cleavage of the terminal 2',3'-cis diol system. Depending on the specific requirements for delivery systems, other possible modifications may include different linker arms containing carboxyl, amino or thiol functionalities. Yet further examples include use of methylphosphonates and 2'-O-methylribose and 5' or 3' capping or blocking with $m_7$GpppG or $m_3^{2,2,7}$GpppG.

For example, a kinased ribozyme ($^{32}$P end labelled using T4 oligonucleotide kinase) is contacted with guanosine triphosphate and guanyltransferase to add a $m^3$G cap to the ribozyme. After such synthesis, the ribozyme can be gel purified using standard procedure. To ensure that the ribozyme has the desired activity, it may be tested with and without the 5' cap using standard procedures to assay both its enzymatic activity and its stability.

Synthetic ribozymes, including those containing various modifiers, can be purified by high pressure liquid chromatography (HPLC). Other liquid chromatography techniques, employing reverse phase columns and anion exchangers on silica and polymeric supports may also be used.

There follows an example of the synthesis of one ribozyme. (See, Dudycz, U.S. Serial No. 07/884,436, filed May 14, 1992, hereby incorporated by reference herein.) A solid phase phosphoramidite chemistry is employed. Monomers used are 2'-tert-butyl-dimethylsilyl cyanoethylphosphoramidities of uridine, N-benzoyl-cytosine, N-phenoxyacetyl adenosine and guanosine (Glen Research, Sterling, VA). Solid phase synthesis is carried out on either an ABI 394 or 380B DNA/RNA synthesizer using the standard protocol provided with each machine. The only exception is that the coupling step is increased from 10 to 12 minutes. The phosphoramidite concentration is 0.1 M. Synthesis is done on a 1 μmole scale using a 1 μmole RNA reaction column (Glen Research). The average coupling efficiencies are between 97% and 98% for the 394 model, and between 97% and 99% for the 380B model, as determined by a calorimetric measurement of the released trityl cation.

Blocked ribozymes are cleaved from the solid support (e.g., CPG), and the bases and diphosphoester moiety deprotected in a sterile vial by dry ethanolic ammonia (2 mL) at 55°C for 16 hours. The reaction mixture is cooled on dry ice. Later, the cold liquid is transferred into a sterile screw cap vial and lyophilized.

To remove the 2'-tert-butyl-dimethylsilyl groups from the ribozyme, the residue is suspended in 1 M tetra-n-butylammonium fluoride in dry THF (TBAF), using a 20 fold excess of the reagent for every silyl group, for 16 hours at ambient temperature (about 15-25°C). The reaction is quenched by adding an equal volume of sterile 1 M triethylamine acetate, pH 6.5. The sample is cooled and concentrated on a SpeedVac to half the initial volume.

The ribozymes are purified in two steps by HPLC on a C4 300 Å 5 mm DeltaPak column in an acetonitrile gradient.

The first step, or "trityl on" step, is a separation of 5'-DMT-protected ribozyme(s) from failure sequences lacking a 5'-DMT group. Solvents used for this step are: A (0.1 M triethylammonium acetate, pH 6.8) and B (acetonitrile). The elution profile is: 20% B for 10 minutes, followed by a linear gradient of 20% B to 50% B over 50 minutes, 50% B for 10 minutes, a linear gradient of 50% B to 100% B over 10 minutes, and a linear gradient of 100% B to 0% B over 10 minutes.

The second step is a purification of a completely deblocked ribozyme by a treatment of 2% trifluoroacetic acid on a C4 300 Å 5 mm DeltaPak column in an acetonitrile gradient. Solvents used for this second step are: A (0.1 M Triethylammonium acetate, pH 6.8) and B (80% acetonitrile, 0.1 M triethylammonium acetate, pH 6.8). The elution profile is: 5% B for 5 minutes, a linear gradient of 5% B to 15% B over 60 minutes, 15% B for 10 minutes, and a linear gradient of 15% B to 0% B over 10 minutes.

The fraction containing ribozyme is cooled and lyophilized on a SpeedVac. Solid residue is dissolved in a minimum amount of ethanol and sodium perchlorate in acetone. The ribozyme is collected by centrifugation, washed three times with acetone, and lyophilized.

Expression Vector

While synthetic ribozymes are preferred in this invention, those produced by expression vectors can also be used. (See, McSwiggen, U.S. Serial No. 07/884,431, filed May 14, 1992, and Draper, U.S. Serial No. 07/923,738, filed July 31, 1992, both hereby incorporated by reference herein.) In designing a suitable ribozyme expression vector the following factors are important to consider. The final ribozyme must be kept as small as possible to minimize unwanted secondary structure within the ribozyme. A promoter (e.g., the human cytomegalovirus immediate early promoter or human beta actin promoter) should be chosen to be a relatively strong promoter, and expressible both in vitro and in vivo (e.g., the human cytomegalovirus immediate early promoter or human beta actin promoter). Such a promoter should express the ribozyme at a level suitable to effect production of enough ribozyme to destroy a target RNA, but not at too high a level to prevent other cellular activities from occurring (unless cell death itself is desired). Useful vectors may also include those based upon Adeno Associated Virus (AAV)

A hairpin at the 5' end of the ribozyme is useful to ensure that the required transcription initiation sequence (GG or GGG or GGGAG) does not bind to some other part of the ribozyme and thus affect regulation of the transcription process. The 5' hairpin is also useful to protect the ribozyme from 5'-3' exonucleases. A selected hairpin at the 3' end of the ribozyme gene is useful since it acts as a transcription termination signal, and protects the ribozyme from 3'-5' exonuclease activity. One example of a known termination signal is that present on the T7 RNA polymerase system. This signal is about 30 nucleotides in length. Other 3' hairpins of shorter length can be used to provide good termination and RNA stability. Such hairpins can be inserted within the vector sequences to allow standard ribozymes to be placed in an appropriate orientation and expressed with such sequences attached.

Poly(A) tails are also useful to protect the 3' end of the ribozyme. These can be provided by either including a poly(A) signal site in the expression vector (to signal a cell to add the poly (A) tail in vivo), or by introducing a poly (A) sequence directly into the expression vector. In the first approach the signal must be located to prevent unwanted secondary structure formation with other parts of the ribozyme. In the second approach, the poly(A) stretch may reduce in size over time when expressed in vivo, and thus the vector may need to be checked over time. Care must be taken in addition of a poly(A) tail which binds poly(A) binding proteins which prevent the ribozyme from acting.

Ribozyme Testing

Once the desired ribozymes are selected, synthesized and purified, they are tested in kinetic and other experiments to determine their utility. An example of such a procedure is provided below.

Preparation of Ribozyme

Crude synthetic ribozyme (typically 350 μg at a time) is purified by separation on a 15% denaturing polyacrylamide gel (0.75 mm thick, 40 cm long) and visualized by UV shadowing. Once excised, gel slices containing full length ribozyme are soaked in 5 ml gel elution buffer (0.5 M NH$_4$OAc, 1 mM EDTA) overnight with shaking at 4°C. The eluent is desalted over a C-18 matrix (Sep-Pak cartridges, Millipore, Milford, MA) and vacuum dried. The dried RNA is resuspended in 50-100 μl TE (TRIS 10 mM, EDTA 1 mM, pH 7.2). An aliquot of this solution is diluted 100 fold into 1 ml TE, half of which is used to spectrophotometrically quantitate the ribozyme solution. The concentration of this dilute stock is typically 150-800 nM. Purity of the ribozyme is confirmed by the presence of a single band on a denaturing polyacrylamide gel. Other equivalent buffers can be used to achieve essentially the same result.

A ribozyme may advantageously be synthesized in two or more portions. (See, Mamone, U.S. Serial No. 07/882,689, filed May 11, 1992, hereby incorporated by reference herein.) Each portion of a ribozyme will generally have only limited or no enzymatic activity, and the activity will increase substantially (by at least 5-10 fold) when all portions are ligated (or otherwise juxtaposed) together. A specific example of hammerhead ribozyme synthesis is provided below.

The method involves synthesis of two (or more) shorter "half" ribozymes and ligation of them together using T4 RNA ligase. For example, to make a 34 mer ribozyme, two 17 mers are synthesized, one is phosphorylated, and both are gel purified. These purified 17 mers are then annealed to a DNA splint strand complementary to the two 17 mers. (Such a splint is not always necessary.) This DNA splint has a sequence designed to locate the two 17 mer portions with one end of each adjacent each other. The juxtaposed RNA molecules are then treated with T4 RNA ligase in the presence of ATP. The 34 mer RNA so formed is then HPLC purified.

Preparation of Substrates

Approximately 10-30 pmoles of unpurified substrate is radioactively 5' end-labeled with T4 polynucleotide kinase using 25 pmoles of [γ-$^{32}$P] ATP. The entire labeling mix is separated on a 20% denaturing polyacrylamide gel and visualized by autoradiography. The full length band is excised and soaked overnight at 4°C in 100 μl of TE (10 mM Tris-HCl pH 7.6, 0.1 mM EDTA).

Kinetic Reactions

For reactions using short substrates (between 8 and 16 bases) a substrate solution is made 1X in assay buffer (75 mM Tris-HCl, pH 7.6; 0.1 mM EDTA, 10 mM MgCl$_2$, or 50 mM Tris-HCl, pH 7.5, 10mM MgCl$_2$) such that the concentration of substrate is less than 1 nM. A ribozyme solution (typically 20 nM) is made 1X in assay buffer and four dilutions are made using 1X assay buffer. Fifteen μl of each ribozyme dilution (i.e., 20, 16, 12, 8 and 4 nM) is placed in a separate tube. These tubes and the substrate tube are pre-incubated at 37°C for at least five minutes.

The reaction is started by mixing 15 μl of substrate into each ribozyme tube by rapid pipetting (note that final ribozyme concentrations are 10, 8, 6, 4, 2 nM). Five μl aliquots are removed at 15 or 30 second intervals and quenched with 5 μl stop solution (95% formamide, 20 mM EDTA xylene cyanol, and bromphenol blue dyes). Following the final ribozyme time point, an aliquot of the remaining substrate is removed as a zero ribozyme control.

The samples are separated on either 15% or 20% polyacrylamide gels. Each gel is visualized and quantitated with an Ambis beta scanner (Ambis Systems, San Diego, CA).

For the most active ribozymes, kinetic analyses are performed in substrate excess to determine $K_m$ and $K_{cat}$ values.

For kinetic reactions with long RNA substrates (greater than 15 bases in length) the substrates are prepared by transcription using T7 RNA polymerase and defined templates containing a T7 promoter, and DNA encoding appropriate nucleotides of the target RNA. The substrate solution is made 1X in assay buffer (75 mM Tris-HCl, pH 7.6; 0.1 mM EDTA; 10 mM MgCl$_2$, or 50 mM Tris-HCl, pH 7.5, 10mM MgCl$_2$) and contains 58 nanomolar concentration of the long RNA molecules. The reaction is started by addition of gel purified ribozymes to 1 μM concentration. Aliquots are removed at 20, 40, 60, 80 and 100 minutes, then quenched by the addition of 5 μl stop solution. Cleavage products are separated using denaturing PAGE. The bands are visualized and quantitated with an Ambis beta scanner or Molecular Dynamics Phosphor Images.

Kinetic Analysis

A simple reaction mechanism for ribozyme-mediated cleavage is:

$$R + S \underset{k_{-1}}{\overset{k_1}{\rightleftarrows}} [R:S] \overset{k_2}{\rightleftarrows} [R:P] \quad \boxed{\rightarrow R + P}$$

where R = ribozyme, S = substrate, and P = products. The boxed step is important only in substrate excess. Because ribozyme concentration is in excess over substrate concentration, the concentration of the ribozyme-substrate complex ([R:S]) is constant over time except during the very brief time when the complex is being initially formed, i.e., :

$$\frac{d[R:S]}{dt} = 0$$

where t = time, and thus:

$$(R)\,(S)k_1 = (RS)\,(k_2 + k_1).$$

The rate of the reaction is the rate of disappearance of substrate with time:

$$Rate = \frac{-d(S)}{dt} = k_2(RS)$$

Substituting these expressions:

$$(R)\,(S)k_1 = 1/k_2 \frac{-d(S)}{dt}\,(k_2 + k_1)$$

or:

$$\frac{-d(S)}{S} = \frac{k_1 k_2}{(k_2 + k_1)}\,(R)\,dt$$

Integrating this expression with respect to time yields:

$$-\ln\frac{S}{S_0} = \frac{k_1 k_2}{(k_2 + k_1)}\,(R)\,t$$

where $S_0$ = initial substrate. Therefore, a plot of the negative log of fraction substrate uncut versus time (in minutes) yields a straight line with slope:

$$slope = \frac{k_1 k_2}{(k_2 + k_1)}\,(R) = k_{obs}$$

where $k_{obs}$ = observed rate constant. A plot of slope ($k_{obs}$) versus ribozyme concentration yields a straight line with a slope which is:

$$slope = \frac{k_1 k_2}{(k_2 + k_1)} \text{ which is } \frac{k_{cat}}{K_m}$$

Using these equations the data obtained from the kinetic experiments provides the necessary information to determine which ribozyme tested is most useful, or active. Such ribozymes can be selected and tested in *in vivo* or *ex*

*vivo* systems.

### Liposome Preparation

Lipid molecules are dissolved in a volatile organic solvent ($CHCl_3$, methanol, diethylether, ethanol, etc.). The organic solvent is removed by evaporation. The lipid is hydrated into suspension with 0.1x phosphate buffered saline (PBS), then freeze-thawed 3x using liquid nitrogen and incubation at room temperature. The suspension is extruded sequentially through a 0.4 µm, 0.2 µm and 0.1 µm polycarbonate filters at maximum pressure of 800 psi. The ribozyme is mixed with the extruded liposome suspension and lyophilized to dryness. The lipid/ribozyme powder is rehydrated with water to one-tenth the original volume. The suspension is diluted to the minimum volume required for extrusion (0.4 ml for 1.5 ml barrel and 1.5 ml for 10 ml barrel) with 1xPBS and re-extruded through 0.4 µm, 0.2 µm, 0.1 µm polycarbonate filters. The liposome entrapped ribozyme is separated from untrapped ribozyme by gel filtration chromatography (SEPHAROSE CL-4B, BIOGEL A5M). The liposome extractions are pooled and sterilized by filtration through a 0.2 µm filter. The free ribozyme is pooled and recovered by ethanol precipitation. The liposome concentration is determined by incorporation of a radioactive lipid. The ribozyme concentration is determined by labeling with $^{32}$P. Rossi et al., 1992, <u>supra</u> (and references cited therein) describe other methods suitable for preparation of liposomes.

### In Vivo Assay

The efficacy of action of a chosen ribozyme may be tested *in vivo* using standard procedures in transformed cells or animals which express the target mRNA.

### Ribonuclease Protection Assay

The accumulation of target mRNA in cells or the cleavage of the RNA by ribozymes or RNAseH (*in vitro* or *in vivo)* can be quantified using an RNAse protection assay. (See, McSwiggen, U.S. Serial Nos. 07/883,849 and 07/884,073, both filed May 14, 1992, hereby incorporated by reference herein.)

In this method, antisense riboprobes are transcribed from template DNA using T7 RNA polymerase (U.S. Biochemicals) in 20 µl reactions containing 1X transcription buffer (supplied by the manufacturer), 0.2 mM ATP, GTP and UTP, 1 U/µl pancreatic RNAse inhibitor (Boehringer Mannheim Biochemicals) and 200 µCi $^{32}$P-labeled CTP (800 Ci/mmol, New England Nuclear) for 1 hour at 37°C. Template DNA is digested with 1 U RNAse-free DNAseI (U.S. Biochemicals, Cleveland, OH) at 37°C for 15 minutes and unincorporated nucleotides removed by G-50 SEPHADEX spin chromatography.

In a manner similar to the transcription of antisense probe, the target RNA can be transcribed *in vitro* using a suitable DNA template. The transcript is purified by standard methods and digested with ribozyme at 37°C according to methods described later.

Alternatively, afflicted (mRNA-expressing) cells are harvested into 1 ml of PBS, transferred to a 1.5 ml EPPENDORF tube, pelleted for 30 seconds at low speed in a microcentrifuge, and lysed in 70 µl of hybridization buffer (4 M guanidine isothiocyanate, 0.1% sarcosyl, 25 mM sodium citrate, pH 7.5). Cell lysate (45 µl) or defined amounts of *in vitro* transcript (also in hybridization buffer) is then combined with 5 µl of hybridization buffer containing 5 x 10$^5$ cpm of each antisense riboprobe in 0.5 ml Eppendorf tubes, overlaid with 25 µl mineral oil, and hybridization accomplished by heating overnight at 55°C (or anywhere between 37°C and 55°C). The hybridization reactions are diluted into 0.5 ml RNAse solution (20 U/ml RNAse A, 2 U/ml RNAse T1, 10 U/ml RNAse-free DNAseI in 0.4 M NaCl), heated for 30 minutes at 37°C, and 10 µl of 20% SDS and 10 µl of Proteinase K (10 mg/ml) added, followed by an additional 30 minutes incubation at 37°C. Hybrids are partially purified by extraction with 0.5 ml of a 1:1 mixture of phenol/chloroform; aqueous phases are combined with 0.5 ml isopropanol, and RNAse-resistant hybrids pelleted for 10 minutes at room temperature (about 20°C) in a microcentrifuge. Pellets are dissolved in 10 µl loading buffer (95% formamide, 1X TBE, 0.1% bromophenol blue, 0.1% xylene cylanol), heated to 95°C for five minutes, cooled on ice, and analyzed on 4% polyacrylamide/7M urea gels under denaturing conditions.

### Ribozyme Stability

The chosen ribozyme can be tested to determine its stability, and thus its potential utility. Such a test can also be used to determine the effect of various chemical modifications (<u>e.g.</u>, addition of a poly(A) tail) on the ribozyme stability and thus aid selection of a more stable ribozyme. For example, a reaction mixture contains 1 to 5 pmoles of 5' (kinased) and/or 3' labeled ribozyme, 15 µg of cytosolic extract and 2.5 mM $MgCl_2$ in a total volume of 100 µl. The reaction is incubated at 37°C. Eight µl aliquots are taken at timed intervals and mixed with 8 µl of a stop mix (20 mM EDTA, 95% formamide). Samples are separated on a 15% acrylamide sequencing gel, exposed to film, and scanned with an Ambis.

A 3'-labeled ribozyme can be formed by incorporation of the $^{32}$P-labeled cordycepin at the 3' OH using poly (A) polymerase. For example, the poly(A) polymerase reaction contains 40 mM Tris, pH 8, 10 mM $MgCl_2$, 250 mM NaCl, 2.5 mM $MnCl_2$,; 3 $\mu$l $^{32}$P cordycepin, 500 Ci/mM; and 6 units poly(A) polymerase in a total volume of 50 $\mu$l. The reaction mixture is incubated for 30 minutes at 37°C.

Effect of Base Substitution Upon Ribozyme Activity

To determine which primary structural characteristics could change ribozyme cleavage of substrate, minor base changes can be made in the substrate cleavage region recognized by a specific ribozyme. For example, the substrate sequences can be changed at the central "C" nucleotide, changing the cleavage site from a GUC to a GUA motif. (See, McSwiggen, U.S. Serial No. 07/884,074, filed May 14, 1992, hereby incorporated by reference herein.) The $K_{cat}/K_m$ values for cleavage using each substrate are then analyzed to determine if such a change increases ribozyme cleavage rates. Similar experiments can be performed to address the effects of changing bases complementary to the ribozyme binding arms. Changes predicted to maintain strong binding to the complementary substrate are preferred. Minor changes in nucleotide content can alter ribozyme/substrate interactions in ways which are unpredictable based upon binding strength alone. Structures in the catalytic core region of the ribozyme recognize trivial changes in either substrate structure or the three dimensional structure of the ribozyme/substrate complex.

To begin optimizing ribozyme design, the cleavage rates of ribozymes containing varied arm lengths, but targeted to the same length of short RNA substrate can be tested. Minimal arm lengths are required and effective cleavage varies with ribozyme/substrate combinations.

The cleavage activity of selected ribozymes can be assessed using target mRNA substrates. The assays are performed in ribozyme excess and approximate $K_{cat}/K_{min}$ values obtained. Comparison of values obtained with short and long substrates indicates utility *in vivo* of a ribozyme.

Intracellular Stability of Liposome-delivered Ribozymes

To test the stability of a chosen ribozyme *in vivo* the following test is useful. Ribozymes are $^{32}$P-end labeled, entrapped in liposomes and delivered to target mRNA-containing cells for three hours. The cells are fractionated and ribozyme is purified by phenol/chloroform extraction. Alternatively, cells ($1 \times 10^7$, T-175 flask) are scraped from the surface of the flask and washed twice with cold PBS. The cells are homogenized by douncing 35 times in 4 ml of TSE (10 mM Tris, pH 7.4, 0.25 M Sucrose, 5 mM EDTA). Nuclei are pelleted at 100xg for 10 minutes. Subcellular organelles (the membrane fraction) are pelleted at 200,000xg for two hours using an SW60 rotor. The pellet is resuspended in 1 ml of H buffer (0.25 M Sucrose, 50 mM HEPES, pH 7.4). The supernatant contains the cytoplasmic fraction (in approximately 3.7 ml). The nuclear pellet is resuspended in 1 ml of 65% sucrose in TM (50 mM Tris, pH 7.4, 2.5 mM $MgCl_2$) and banded on a sucrose step gradient (1 ml nuclei in 65% sucrose TM, 1 ml 60% sucrose TM, 1 ml 55% sucrose TM, 50% sucrose TM, 300 $\mu$l 25% sucrose TM) for one hour at 37,000xg with an SW60 rotor. The nuclear band is harvested and diluted to 10% sucrose with TM buffer. Nuclei are pelleted at 37,000xg using an SW60 rotor for 15 minutes and the pellet resuspended in 1 ml of TM buffer. Aliquots are size fractionated on denaturing polyacrylamide gels and the intracellular localization determined. By comparison to the migration rate of newly synthesized ribozyme, the various fractions containing intact ribozyme can be determined.

To investigate modifications which would lengthen the half-life of ribozyme molecules intracellularly, the cells may be fractioned as above and the purity of each fraction assessed by assaying enzyme activity known to exist in that fraction.

The various cell fractions are frozen at -70°C and used to determine relative nuclease resistances of modified ribozyme molecules. Ribozyme molecules may be synthesized with 5 phosphorothioate (ps), or 2'-Omethyl (2'-OMe) modifications at each end of the molecule. These molecules and a phosphodiester version of the ribozyme are end-labeled with $^{32}$P and ATP using T4 polynucleotide kinase. Equal concentrations are added to the cell cytoplasmic extracts and aliquots of each taken at 10 minute intervals. The samples are size fractionated by denaturing PAGE and relative rates of nuclease resistance analyzed by scanning the gel with an Ambis $\beta$-scanner. The results show whether the ribozymes are digested by the cytoplasmic extract, and which versions are relatively more nuclease resistant. Modified ribozymes generally maintain 80-90% of the catalytic activity of the native ribozyme when short RNA substrates are employed.

Unlabeled, 5' end-labeled or 3' end-labeled ribozymes can be used in the assays. These experiments can also be performed with human cell extracts to verify the observations.

The following are non-limiting examples of the present invention.

Example 1: Ribozyme target sites in human stromelysin mRNA

At present, hammerhead ribozyme target sites are constrained only to RNA sequences which contain a U followed by any nucleotide except a G, i.e. UC, UA and UU. To locate potential hammerhead ribozyme target sites in the human stromelysin mRNA sequence, the entire RNA sequence (1801 nucleotides) was examined for UH sequences and 326 sites were found. These 326 potential sites and their immediate flanking sequences are listed in Table 2. In addition to all UH sites, Table 2 includes 8 potential hairpin ribozyme target sites in stromelysin RNA. These are SEQ. ID. NOS.: 360-367.

The position number refers to the nucleotide 5' to the potential ribozyme cleavage site.

```
                           Table_2
    Input Sequence  = HUMSTROM.RNA;  Cut Site = UH.
    Stem Length =  15
    Position Target Sequence              Seq. ID. NO.
       10     GCAAGGCAUA GAGACAACAUAGAGC      ID. NO. 34
       21     GCAUAGAGACAACAUA GAGCUAAGUAAAGCC  ID. NO. 35
       27     AGACAACAUAGAGCUA AGUAAAGCCAGUGGA  ID. NO. 36
       31     AACAUAGAGCUAAGUA AAGCCAGUGGAAAUG  ID. NO. 37
       53     GUGGAAAUGAAGAGUC UUCCAAUCCUACUGU  ID. NO. 38
```

| 55 | GGAAAUGAAGAGUCUU CCAAUCCUACUGUUG | ID. NO. 39 |
|---|---|---|
| 56 | GAAAUGAAGAGUCUUC CAAUCCUACUGUUGC | ID. NO. 40 |
| 61 | GAAGAGUCUUCCAAUC CUACUGUUGCUGUGC | ID. NO. 41 |
| 64 | GAGUCUUCCAAUCCUA CUGUUGCUGUGCGUG | ID. NO. 42 |
| 69 | UUCCAAUCCUACUGUU GCUGUGCGUGGCAGU | ID. NO. 43 |
| 85 | GCUGUGCGUGGCAGUU UGCUCAGCCUAUCCA | ID. NO. 44 |
| 86 | CUGUGCGUGGCAGUUU GCUCAGCCUAUCCAU | ID. NO. 45 |
| 90 | GCGUGGCAGUUUGCUC AGCCUAUCCAUUGGA | ID. NO. 46 |
| 96 | CAGUUUGCUCAGCCUA UCCAUUGGAUGGAGC | ID. NO. 47 |
| 98 | GUUUGCUCAGCCUAUC CAUUGGAUGGAGCUG | ID. NO. 48 |
| 102 | GCUCAGCCUAUCCAUU GGAUGGAGCUGCAAG | ID. NO. 49 |
| 142 | CACCAGCAUGAACCUU GUUCAGAAAUAUCUA | ID. NO. 50 |
| 145 | CAGCAUGAACCUUGUU CAGAAAUAUCUAGAA | ID. NO. 51 |
| 146 | AGCAUGAACCUUGUUC AGAAAUAUCUAGAAA | ID. NO. 52 |
| 153 | ACCUUGUUCAGAAAUA UCUAGAAAACUACUA | ID. NO. 53 |
| 155 | CUUGUUCAGAAAUAUC UAGAAAACUACUACG | ID. NO. 54 |
| 157 | UGUUCAGAAAUAUCUA GAAAACUACUACGAC | ID. NO. 55 |
| 165 | AAUAUCUAGAAAACUA CUACGACCUCAAAAA | ID. NO. 56 |
| 168 | AUCUAGAAAACUACUA CGACCUCAAAAAAGA | ID. NO. 57 |
| 175 | AAACUACUACGACCUC AAAAAAGAUGUGAAA | ID. NO. 58 |
| 195 | AAGAUGUGAAACAGUU UGUUAGGAGAAAGGA | ID. NO. 59 |
| 196 | AGAUGUGAAACAGUUU GUUAGGAGAAAGGAC | ID. NO. 60 |
| 199 | UGUGAAACAGUUUGUU AGGAGAAAGGACAGU | ID. NO. 61 |
| 200 | GUGAAACAGUUUGUUA GGAGAAAGGACAGUG | ID. NO. 62 |
| 218 | AGAAAGGACAGUGGUC CUGUUGUUAAAAAAA | ID. NO. 63 |
| 223 | GGACAGUGGUCCUGUU GUUAAAAAAAUCCGA | ID. NO. 64 |
| 226 | CAGUGGUCCUGUUGUU AAAAAAAUCCGAGAA | ID. NO. 65 |
| 227 | AGUGGUCCUGUUGUUA AAAAAAUCCGAGAAA | ID. NO. 66 |
| 235 | UGUUGUUAAAAAAAUC CGAGAAAUGCAGAAG | ID. NO. 67 |
| 252 | GAGAAAUGCAGAAGUU CCUUGGAUUGGAGGU | ID. NO. 68 |
| 253 | AGAAAUGCAGAAGUUC CUUGGAUUGGAGGUG | ID. NO. 69 |
| 256 | AAUGCAGAAGUUCCUU GGAUUGGAGGUGACG | ID. NO. 70 |
| 261 | AGAAGUUCCUUGGAUU GGAGGUGACGGGGAA | ID. NO. 71 |
| 285 | CGGGGAAGCUGGACUC CGACACUCUGGAGGU | ID. NO. 72 |
| 293 | CUGGACUCCGACACUC UGGAGGUGAUGCGCA | ID. NO. 73 |
| 325 | GCCCAGGUGUGGAGUU CCUGAUGUUGGUCAC | ID. NO. 74 |
| 326 | CCCAGGUGUGGAGUUC CUGAUGUUGGUCACU | ID. NO. 75 |

334    UGGAGUUCCUGAUGUU GGUCACUUCAGAACC    ID. NO. 76

338    GUUCCUGAUGUUGGUC ACUUCAGAACCUUUC    ID. NO. 77

342    CUGAUGUUGGUCACUU CAGAACCUUUCCUGG    ID. NO. 78

343    UGAUGUUGGUCACUUC AGAACCUUUCCUGGC    ID. NO. 79

351    GUCACUUCAGAACCUU UCCUGGCAUCCCGAA    ID. NO. 80

352    UCACUUCAGAACCUUU CCUGGCAUCCCGAAG    ID. NO. 81

353    CACUUCAGAACCUUUC CUGGCAUCCCGAAGU    ID. NO. 82

361    AACCUUUCCUGGCAUC CCGAAGUGGAGGAAA    ID. NO. 83

385    GAGGAAAACCCACCUU ACAUACAGGAUUGUG    ID. NO. 84

386    AGGAAAACCCACCUUA CAUACAGGAUUGUGA    ID. NO. 85

390    AAACCCACCUUACAUA CAGGAUUGUGAAUUA    ID. NO. 86

397    CCUUACAUACAGGAUU GUGAAUUAUACACCA    ID. NO. 87

404    UACAGGAUUGUGAAUU AUACACCAGAUUUGC    ID. NO. 88

405    ACAGGAUUGUGAAUUA UACACCAGAUUUGCC    ID. NO. 89

407    AGGAUUGUGAAUUAUA CACCAGAUUUGCCAA    ID. NO. 90

416    AAUUAUACACCAGAUU UGCCAAAAGAUGCUG    ID. NO. 91

417    AUUAUACACCAGAUUU GCCAAAAGAUGCUGU    ID. NO. 92

433    GCCAAAAGAUGCUGUU GAUUCUGCUGUUGAG    ID. NO. 93

437    AAAGAUGCUGUUGAUU CUGCUGUUGAGAAAG    ID. NO. 94

438    AAGAUGCUGUUGAUUC UGCUGUUGAGAAAGC    ID. NO. 95

445    UGUUGAUUCUGCUGUU GAGAAAGCUCUGAAA    ID. NO. 96

455    GCUGUUGAGAAAGCUC UGAAAGUCUGGGAAG    ID. NO. 97

463    GAAAGCUCUGAAAGUC UGGGAAGAGGUGACU    ID. NO. 98

479    UGGGAAGAGGUGACUC CACUCACAUUCUCCA    ID. NO. 99

484    AGAGGUGACUCCACUC ACAUUCUCCAGGCUG    ID. NO. 100

489    UGACUCCACUCACAUU CUCCAGGCUGUAUGA    ID. NO. 101

490    GACUCCACUCACAUUC UCCAGGCUGUAUGAA    ID. NO. 102

492    CUCCACUCACAUUCUC CAGGCUGUAUGAAGG    ID. NO. 103

501    CAUUCUCCAGGCUGUA UGAAGGAGAGGCUGA    ID. NO. 104

518    GAAGGAGAGGCUGAUA UAAUGAUCUCUUUUG    ID. NO. 105

520    AGGAGAGGCUGAUAUA AUGAUCUCUUUUGCA    ID. NO. 106

526    GGCUGAUAUAAUGAUC UCUUUUGCAGUUAGA    ID. NO. 107

528    CUGAUAUAAUGAUCUC UUUUGCAGUUAGAGA    ID. NO. 108

530    GAUAUAAUGAUCUCUU UUGCAGUUAGAGAAC    ID. NO. 109

531    AUAUAAUGAUCUCUUU UGCAGUUAGAGAACA    ID. NO. 110

532    UAUAAUGAUCUCUUUU GCAGUUAGAGAACAU    ID. NO. 111

538    GAUCUCUUUUGCAGUU AGAGAACAUGGAGAC    ID. NO. 112

```
539    AUCUCUUUUGCAGUUA GAGAACAUGGAGACU   ID. NO. 113
555    GAGAACAUGGAGACUU UUACCCUUUUGAUGG    ID. NO. 114
556    AGAACAUGGAGACUUU UACCCUUUUGAUGGA    ID. NO. 115
557    GAACAUGGAGACUUUU ACCCUUUUGAUGGAC    ID. NO. 116
558    AACAUGGAGACUUUUA CCCUUUUGAUGGACC    ID. NO. 117
563    GGAGACUUUUACCCUU UUGAUGGACCUGGAA    ID. NO. 118
564    GAGACUUUUACCCUUU UGAUGGACCUGGAAA    ID. NO. 119
565    AGACUUUUACCCUUUU GAUGGACCUGGAAAU    ID. NO. 120
583    UGGACCUGGAAAUGUU UUGGCCCAUGCCUAU    ID. NO. 121
584    GGACCUGGAAAUGUUU UGGCCCAUGCCUAUG    ID. NO. 122
585    GACCUGGAAAUGUUUU GGCCCAUGCCUAUGC    ID. NO. 123
597    UUUUGGCCCAUGCCUA UGCCCCUGGGCCAGG    ID. NO. 124
616    CCCUGGGCCAGGGAUU AAUGGAGAUGCCCAC    ID. NO. 125
617    CCUGGGCCAGGGAUUA AUGGAGAUGCCCACU    ID. NO. 126
633    AUGGAGAUGCCCACUU UGAUGAUGAUGAACA    ID. NO. 127
634    UGGAGAUGCCCACUUU GAUGAUGAUGAACAA    ID. NO. 128
662    CAAUGGACAAAGGAUA CAACAGGGACCAAUU    ID. NO. 129
677    ACAACAGGGACCAAUU UAUUUCUCGUUGCUG    ID. NO. 130
678    CAACAGGGACCAAUUU AUUUCUCGUUGCUGC    ID. NO. 131
679    AACAGGGACCAAUUUA UUUCUCGUUGCUGCU    ID. NO. 132
681    CAGGGACCAAUUUAUU UCUCGUUGCUGCUCA    ID. NO. 133
682    AGGGACCAAUUUAUUU CUCGUUGCUGCUCAU    ID. NO. 134
683    GGGACCAAUUUAUUUC UCGUUGCUGCUCAUG    ID. NO. 135
685    GACCAAUUUAUUUCUC GUUGCUGCUCAUGAA    ID. NO. 136
688    CAAUUUAUUUCUCGUU GCUGCUCAUGAAAUU    ID. NO. 137
695    UUUCUCGUUGCUGCUC AUGAAAUUGGCCACU    ID. NO. 138
703    UGCUGCUCAUGAAAUU GGCCACUCCCUGGGU    ID. NO. 139
711    AUGAAAUUGGCCACUC CCUGGGUCUCUUUCA    ID. NO. 140
719    GGCCACUCCCUGGGUC UCUUUCACUCAGCCA    ID. NO. 141
721    CCACUCCCUGGGUCUC UUUCACUCAGCCAAC    ID. NO. 142
723    ACUCCCUGGGUCUCUU UCACUCAGCCAACAC    ID. NO. 143
724    CUCCCUGGGUCUCUUU CACUCAGCCAACACU    ID. NO. 144
725    UCCCUGGGUCUCUUUC ACUCAGCCAACACUG    ID. NO. 145
729    UGGGUCUCUUUCACUC AGCCAACACUGAAGC    ID. NO. 146
746    GCCAACACUGAAGCUU UGAUGUACCCACUCU    ID. NO. 147
747    CCAACACUGAAGCUUU GAUGUACCCACUCUA    ID. NO. 148
753    CUGAAGCUUUGAUGUA CCCACUCUAUCACUC    ID. NO. 149
```

760 UUUGAUGUACCCACUC UAUCACUCACUCACA ID. NO. 150

762 UGAUGUACCCACUCUA UCACUCACUCACAGA ID. NO. 151

764 AUGUACCCACUCUAUC ACUCACUCACAGACC ID. NO. 152

768 ACCCACUCUAUCACUC ACUCACAGACCUGAC ID. NO. 153

772 ACUCUAUCACUCACUC ACAGACCUGACUCGG ID. NO. 154

785 CUCACAGACCUGACUC GGUUCCGCCUGUCUC ID. NO. 155

789 CAGACCUGACUCGGUU CCGCCUGUCUCAAGA ID. NO. 156

790 AGACCUGACUCGGUUC CGCCUGUCUCAAGAU ID. NO. 157

798 CUCGGUUCCGCCUGUC UCAAGAUGAUAUAAA ID. NO. 158

800 CGGUUCCGCCUGUCUC AAGAUGAUAUAAAUG ID. NO. 159

809 CUGUCUCAAGAUGAUA UAAAUGGCAUUCAGU ID. NO. 160

811 GUCUCAAGAUGAUAUA AAUGGCAUUCAGUCC ID. NO. 161

820 UGAUAUAAAUGGCAUU CAGUCCUCUAUGGA ID. NO. 162

821 GAUAUAAAUGGCAUUC AGUCCUCUAUGGAC ID. NO. 163

825 UAAAUGGCAUUCAGUC CUCUAUGGACCUCC ID. NO. 164

829 UGGCAUUCAGUCCCUC UAUGGACCUCCCCCU ID. NO. 165

831 GCAUUCAGUCCCUCUA UGGACCUCCCCCUGA ID. NO. 166

839 UCCCUCUAUGGACCUC CCCCUGACUCCCCUG ID. NO. 167

849 GACCUCCCCCUGACUC CCCUGAGACCCCCCU ID. NO. 168

868 UGAGACCCCCCUGGUA CCCACGGAACCUGUC ID. NO. 169

883 ACCCACGGAACCUGUC CCUCCAGAACCUGGG ID. NO. 170

887 ACGGAACCUGUCCCUC CAGAACCUGGGACGC ID. NO. 171

917 CCAGCCAACUGUGAUC CUGCUUUGUCCUUUG ID. NO. 172

923 AACUGUGAUCCUGCUU UGUCCUUUGAUGCUG ID. NO. 173

924 ACUGUGAUCCUGCUUU GUCCUUUGAUGCUGU ID. NO. 174

927 GUGAUCCUGCUUUGUC CUUUGAUGCUGUCAG ID. NO. 175

930 AUCCUGCUUUGUCCUU UGAUGCUGUCAGCAC ID. NO. 176

931 UCCUGCUUUGUCCUUU GAUGCUGUCAGCACU ID. NO. 177

940 GUCCUUUGAUGCUGUC AGCACUCUGAGGGGA ID. NO. 178

947 GAUGCUGUCAGCACUC UGAGGGGAGAAAUCC ID. NO. 179

961 UCUGAGGGGAGAAAUC CUGAUCUUUAAAGAC ID. NO. 180

967 GGGAGAAAUCCUGAUC UUUAAAGACAGGCAC ID. NO. 181

969 GAGAAAUCCUGAUCUU UAAAGACAGGCACUU ID. NO. 182

970 AGAAAUCCUGAUCUUU AAAGACAGGCACUUU ID. NO. 183

971 GAAAUCCUGAUCUUUA AAGACAGGCACUUUU ID. NO. 184

984 UUAAAGACAGGCACUU UUGGCGCAAAUCCCU ID. NO. 185

985 UAAAGACAGGCACUUU UGGCGCAAAUCCCUC ID. NO. 186

| | | |
|---|---|---|
| 986 | AAAGACAGGCACUUUU GGCGCAAAUCCCUCA | ID. NO. 187 |
| 996 | ACUUUUGGCGCAAAUC CCUCAGGAAGCUUGA | ID. NO. 188 |
| 1000 | UUGGCGCAAAUCCCUC AGGAAGCUUGAACCU | ID. NO. 189 |
| 1009 | AUCCCUCAGGAAGCUU GAACCUGAAUUGCAU | ID. NO. 190 |
| 1020 | AGCUUGAACCUGAAUU GCAUUUGAUCUCUUC | ID. NO. 191 |
| 1025 | GAACCUGAAUUGCAUU UGAUCUCUUCAUUUU | ID. NO. 192 |
| 1026 | AACCUGAAUUGCAUUU GAUCUCUUCAUUUUG | ID. NO. 193 |
| 1030 | UGAAUUGCAUUUGAUC UCUUCAUUUUGGCCA | ID. NO. 194 |
| 1032 | AAUUGCAUUUGAUCUC UUCAUUUUGGCCAUC | ID. NO. 195 |
| 1034 | UUGCAUUUGAUCUCUU CAUUUUGGCCAUCUC | ID. NO. 196 |
| 1035 | UGCAUUUGAUCUCUUC AUUUUGGCCAUCUCU | ID. NO. 197 |
| 1038 | AUUUGAUCUCUUCAUU UUGGCCAUCUCUUCC | ID. NO. 198 |
| 1039 | UUUGAUCUCUUCAUUU UGGCCAUCUCUUCCU | ID. NO. 199 |
| 1040 | UUGAUCUCUUCAUUUU GGCCAUCUCUUCCUU | ID. NO. 200 |
| 1047 | CUUCAUUUUGGCCAUC UCUUCCUUCAGGCGU | ID. NO. 201 |
| 1049 | UCAUUUUGGCCAUCUC UUCCUUCAGGCGUGG | ID. NO. 202 |
| 1051 | AUUUUGGCCAUCUCUU CCUUCAGGCGUGGAU | ID. NO. 203 |
| 1052 | UUUUGGCCAUCUCUUC CUUCAGGCGUGGAUG | ID. NO. 204 |
| 1055 | UGGCCAUCUCUUCCUU CAGGCGUGGAUGCCG | ID. NO. 205 |
| 1056 | GGCCAUCUCUUCCUUC AGGCGUGGAUGCCGC | ID. NO. 206 |
| 1074 | GCGUGGAUGCCGCAUA UGAAGUUACUAGCAA | ID. NO. 207 |
| 1081 | UGCCGCAUAUGAAGUU ACUAGCAAGGACCUC | ID. NO. 208 |
| 1082 | GCCGCAUAUGAAGUUA CUAGCAAGGACCUCG | ID. NO. 209 |
| 1085 | GCAUAUGAAGUUACUA GCAAGGACCUCGUUU | ID. NO. 210 |
| 1096 | UACUAGCAAGGACCUC GUUUUCAUUUUUAAA | ID. NO. 211 |
| 1099 | UAGCAAGGACCUCGUU UUCAUUUUUAAAGGA | ID. NO. 212 |
| 1100 | AGCAAGGACCUCGUUU UCAUUUUUAAAGGAA | ID. NO. 213 |
| 1101 | GCAAGGACCUCGUUUU CAUUUUUAAAGGAAA | ID. NO. 214 |
| 1102 | CAAGGACCUCGUUUUC AUUUUUAAAGGAAAU | ID. NO. 215 |
| 1105 | GGACCUCGUUUUCAUU UUUAAAGGAAAUCAA | ID. NO. 216 |
| 1106 | GACCUCGUUUUCAUUU UUAAAGGAAAUCAAU | ID. NO. 217 |
| 1107 | ACCUCGUUUUCAUUUU UAAAGGAAAUCAAUU | ID. NO. 218 |
| 1108 | CCUCGUUUUCAUUUUU AAAGGAAAUCAAUUC | ID. NO. 219 |
| 1109 | CUCGUUUUCAUUUUUA AAGGAAAUCAAUUCU | ID. NO. 220 |
| 1118 | AUUUUUAAAGGAAAUC AAUUCUGGGCCAUCA | ID. NO. 221 |
| 1122 | UUAAAGGAAAUCAAUU CUGGGCCAUCAGAGG | ID. NO. 222 |
| 1123 | UAAAGGAAAUCAAUUC UGGGCCAUCAGAGGA | ID. NO. 223 |

| 1132 | UCAAUUCUGGGCCAUC | AGAGGAAAUGAGGUA | ID. NO. 224 |
|------|------------------|-----------------|-------------|
| 1147 | CAGAGGAAAUGAGGUA | CGAGCUGGAUACCCA | ID. NO. 225 |
| 1158 | AGGUACGAGCUGGAUA | CCCAAGAGGCAUCCA | ID. NO. 226 |
| 1171 | AUACCCAAGAGGCAUC | CACACCCUAGGUUUC | ID. NO. 227 |
| 1180 | AGGCAUCCACACCCUA | GGUUUCCCUCCAACC | ID. NO. 228 |
| 1184 | AUCCACACCCUAGGUU | UCCCUCCAACCGUGA | ID. NO. 229 |
| 1185 | UCCACACCCUAGGUUU | CCCUCCAACCGUGAG | ID. NO. 230 |
| 1186 | CCACACCCUAGGUUUC | CCUCCAACCGUGAGG | ID. NO. 231 |
| 1190 | ACCCUAGGUUUCCCUC | CAACCGUGAGGAAAA | ID. NO. 232 |
| 1207 | AACCGUGAGGAAAAUC | GAUGCAGCCAUUUCU | ID. NO. 233 |
| 1219 | AAUCGAUGCAGCCAUU | UCUGAUAAGGAAAAG | ID. NO. 234 |
| 1220 | AUCGAUGCAGCCAUUU | CUGAUAAGGAAAAGA | ID. NO. 235 |
| 1221 | UCGAUGCAGCCAUUUC | UGAUAAGGAAAAGAA | ID. NO. 236 |
| 1226 | GCAGCCAUUUCUGAUA | AGGAAAAGAACAAAA | ID. NO. 237 |
| 1245 | AAAAGAACAAAACAUA | UUUCUUUGUAGAGGA | ID. NO. 238 |
| 1247 | AAGAACAAAACAUAUU | UCUUUGUAGAGGACA | ID. NO. 239 |
| 1248 | AGAACAAAACAUAUUU | CUUUGUAGAGGACAA | ID. NO. 240 |
| 1249 | GAACAAAACAUAUUUC | UUUGUAGAGGACAAA | ID. NO. 241 |
| 1251 | ACAAAACAUAUUUCUU | UGUAGAGGACAAAUA | ID. NO. 242 |
| 1252 | CAAAACAUAUUUCUUU | GUAGAGGACAAAUAC | ID. NO. 243 |
| 1255 | AACAUAUUUCUUUGUA | GAGGACAAAUACUGG | ID. NO. 244 |
| 1266 | UUGUAGAGGACAAAUA | CUGGAGAUUUGAUGA | ID. NO. 245 |
| 1275 | ACAAAUACUGGAGAUU | UGAUGAGAAGAGAAA | ID. NO. 246 |
| 1276 | CAAAUACUGGAGAUUU | GAUGAGAAGAGAAAU | ID. NO. 247 |
| 1292 | GAUGAGAAGAGAAAUU | CCAUGGAGCCAGGCU | ID. NO. 248 |
| 1293 | AUGAGAAGAGAAAUUC | CAUGGAGCCAGGCUU | ID. NO. 249 |
| 1308 | CCAUGGAGCCAGGCUU | UCCCAAGCAAAUAGC | ID. NO. 250 |
| 1309 | CAUGGAGCCAGGCUUU | CCCAAGCAAAUAGCU | ID. NO. 251 |
| 1310 | AUGGAGCCAGGCUUUC | CCAAGCAAAUAGCUG | ID. NO. 252 |
| 1321 | CUUUCCCAAGCAAAUA | GCUGAAGACUUUCCA | ID. NO. 253 |
| 1332 | AAAUAGCUGAAGACUU | UCCAGGGAUUGACUC | ID. NO. 254 |
| 1333 | AAUAGCUGAAGACUUU | CCAGGGAUUGACUCA | ID. NO. 255 |
| 1334 | AUAGCUGAAGACUUUC | CAGGGAUUGACUCAA | ID. NO. 256 |
| 1342 | AGACUUUCCAGGGAUU | GACUCAAAGAUUGAU | ID. NO. 257 |
| 1347 | UUCCAGGGAUUGACUC | AAAGAUUGAUGCUGU | ID. NO. 258 |
| 1354 | GAUUGACUCAAAGAUU | GAUGCUGUUUUUGAA | ID. NO. 259 |
| 1363 | AAAGAUUGAUGCUGUU | UUUGAAGAAUUUGGG | ID. NO. 260 |

| 1364 | AAGAUUGAUGCUGUUU | UUGAAGAAUUUGGGU | ID. NO. 261 |
| 1365 | AGAUUGAUGCUGUUUU | UGAAGAAUUUGGGUU | ID. NO. 262 |
| 1366 | GAUUGAUGCUGUUUUU | GAAGAAUUUGGGUUC | ID. NO. 263 |
| 1374 | CUGUUUUUGAAGAAUU | UGGGUUCUUUUAUUU | ID. NO. 264 |
| 1375 | UGUUUUUGAAGAAUUU | GGGUUCUUUUAUUUC | ID. NO. 265 |
| 1380 | UUGAAGAAUUUGGGUU | CUUUUAUUUCUUUAC | ID. NO. 266 |
| 1381 | UGAAGAAUUUGGGUUC | UUUUAUUUCUUUACU | ID. NO. 267 |
| 1383 | AAGAAUUUGGGUUCUU | UUAUUUCUUUACUGG | ID. NO. 268 |
| 1384 | A  AUUUGGGUUCUUU | UAUUUCUUUACUGGA | ID. NO. 269 |
| 1385 | G  UUUGGGUUCUUUU | A  UUCUUUACUGGAU | ID. NO. 270 |
| 1386 | AAUUUGGGUUCUUUUA | UUUCUUUACUGGAUC | ID. NO. 271 |
| 1388 | UUUGGGUUCUUUUAUU | UCUUUACUGGAUCUU | ID. NO. 272 |
| 1389 | UUGGGUUCUUUUAUUU | CUUUACUGGAUCUUC | ID. NO. 273 |
| 1390 | UGGGUUCUUUUAUUUC | UUUACUGGAUCUUCA | ID. NO. 274 |
| 1392 | GGUUCUUUUAUUUCUU | UACUGGAUCUUCACA | ID. NO. 275 |
| 1393 | GUUCUUUUAUUUCUUU | ACUGGAUCUUCACAG | ID. NO. 276 |
| 1394 | UUCUUUUAUUUCUUUA | CUGGAUCUUCACAGU | ID. NO. 277 |
| 1401 | AUUUCUUUACUGGAUC | UUCACAGUUGGAGUU | ID. NO. 278 |
| 1403 | UUCUUUACUGGAUCUU | CACAGUUGGAGUUUG | ID. NO. 279 |
| 1404 | UCUUUACUGGAUCUUC | ACAGUUGGAGUUUGA | ID. NO. 280 |
| 1410 | CUGGAUCUUCACAGUU | GGAGUUUGACCCAAA | ID. NO. 281 |
| 1416 | CUUCACAGUUGGAGUU | UGACCCAAAUGCAAA | ID. NO. 282 |
| 1417 | UUCACAGUUGGAGUUU | GACCCAAAUGCAAAG | ID. NO. 283 |
| 1448 | AAAGUGACACACACUU | UGAAGAGUAACAGCU | ID. NO. 284 |
| 1449 | AAGUGACACACACUUU | GAAGAGUAACAGCUG | ID. NO. 285 |
| 1457 | CACACUUUGAAGAGUA | ACAGCUGGCUUAAUU | ID. NO. 286 |
| 1468 | GAGUAACAGCUGGCUU | AAUUGUUGAAAGAGA | ID. NO. 287 |
| 1469 | AGUAACAGCUGGCUUA | AUUGUUGAAAGAGAU | ID. NO. 288 |
| 1472 | AACAGCUGGCUUAAUU | GUUGAAAGAGAUAUG | ID. NO. 289 |
| 1475 | AGCUGGCUUAAUUGUU | GAAAGAGAUAUGUAG | ID. NO. 290 |
| 1485 | AUUGUUGAAAGAGAUA | UGUAGAAGGCACAAU | ID. NO. 291 |
| 1489 | UUGAAAGAGAUAUGUA | GAAGGCACAAUAUGG | ID. NO. 292 |
| 1501 | UGUAGAAGGCACAAUA | UGGGCACUUUAAAUG | ID. NO. 293 |
| 1510 | CACAAUAUGGGCACUU | UAAAUGAAGCUAAUA | ID. NO. 294 |
| 1511 | ACAAUAUGGGCACUUU | AAAUGAAGCUAAUAA | ID. NO. 295 |
| 1512 | CAAUAUGGGCACUUUA | AAUGAAGCUAAUAAU | ID. NO. 296 |
| 1522 | ACUUUAAAUGAAGCUA | AUAAUUCUUCACCUA | ID. NO. 297 |

| 1525 | UUAAAUGAAGCUAAUA AUUCUUCACCUAAGU | ID. NO. 298 |
| 1528 | AAUGAAGCUAAUAAUU CUUCACCUAAGUCUC | ID. NO. 299 |
| 1529 | AUGAAGCUAAUAAUUC UUCACCUAAGUCUCU | ID. NO. 300 |
| 1531 | GAAGCUAAUAAUUCUU CACCUAAGUCUCUGU | ID. NO. 301 |
| 1532 | AAGCUAAUAAUUCUUC ACCUAAGUCUCUGUG | ID. NO. 302 |
| 1537 | AAUAAUUCUUCACCUA AGUCUCUGUGAAUUG | ID. NO. 303 |
| 1541 | AUUCUUCACCUAAGUC UCUGUGAAUUGAAAU | ID. NO. 304 |
| 1543 | UCUUCACCUAAGUCUC UGUGAAUUGAAAUGU | ID. NO. 305 |
| 1551 | UAAGUCUCUGUGAAUU GAAAUGUUCGUUUUC | ID. NO. 306 |
| 1559 | UGUGAAUUGAAAUGUU CGUUUUCUCCUGCCU | ID. NO. 307 |
| 1560 | GUGAAUUGAAAUGUUC GUUUUCUCCUGCCUG | ID. NO. 308 |
| 1563 | AAUUGAAAUGUUCGUU UUCUCCUGCCUGUGC | ID. NO. 309 |
| 1564 | AUUGAAAUGUUCGUUU UCUCCUGCCUGUGCU | ID. NO. 310 |
| 1565 | UUGAAAUGUUCGUUUU CUCCUGCCUGUGCUG | ID. NO. 311 |
| 1566 | UGAAAUGUUCGUUUUC UCCUGCCUGUGCUGU | ID. NO. 312 |
| 1568 | AAAUGUUCGUUUUCUC CUGCCUGUGCUGUGA | ID. NO. 313 |
| 1586 | GCCUGUGCUGUGACUC GAGUCACACUCAAGG | ID. NO. 314 |
| 1591 | UGCUGUGACUCGAGUC ACACUCAAGGGAACU | ID. NO. 315 |
| 1597 | GACUCGAGUCACACUC AAGGGAACUUGAGCG | ID. NO. 316 |
| 1607 | ACACUCAAGGGAACUU GAGCGUGAAUCUGUA | ID. NO. 317 |
| 1618 | AACUUGAGCGUGAAUC UGUAUCUUGCCGGUC | ID. NO. 318 |
| 1622 | UGAGCGUGAAUCUGUA UCUUGCCGGUCAUUU | ID. NO. 319 |
| 1624 | AGCGUGAAUCUGUAUC UUGCCGGUCAUUUUU | ID. NO. 320 |
| 1626 | CGUGAAUCUGUAUCUU GCCGGUCAUUUUUAU | ID. NO. 321 |
| 1633 | CUGUAUCUUGCCGGUC AUUUUUAUGUUAUUA | ID. NO. 322 |
| 1636 | UAUCUUGCCGGUCAUU UUUAUGUUAUUACAG | ID. NO. 323 |
| 1637 | AUCUUGCCGGUCAUUU UUAUGUUAUUACAGG | ID. NO. 324 |
| 1638 | UCUUGCCGGUCAUUUU UAUGUUAUUACAGGG | ID. NO. 325 |
| 1639 | CUUGCCGGUCAUUUUU AUGUUAUUACAGGGC | ID. NO. 326 |
| 1640 | UUGCCGGUCAUUUUUA UGUUAUUACAGGGCA | ID. NO. 327 |
| 1644 | CGGUCAUUUUUAUGUU AUUACAGGGCAUUCA | ID. NO. 328 |
| 1645 | GGUCAUUUUUAUGUUA UUACAGGGCAUUCAA | ID. NO. 329 |
| 1647 | UCAUUUUUAUGUUAUU ACAGGGCAUUCAAAU | ID. NO. 330 |
| 1648 | CAUUUUUAUGUUAUUA CAGGGCAUUCAAAUG | ID. NO. 331 |
| 1657 | GUUAUUACAGGGCAUU CAAAUGGGCUGCUGC | ID. NO. 332 |
| 1658 | UUAUUACAGGGCAUUC AAAUGGGCUGCUGCU | ID. NO. 333 |
| 1674 | AAAUGGGCUGCUGCUU AGCUUGCACCUUGUC | ID. NO. 334 |

| 1675 | AAUGGGCUGCUGCUUA GCUUGCACCUUGUCA | ID. NO. 335 |
| 1679 | GGCUGCUGCUUAGCUU GCACCUUGUCACAUA | ID. NO. 336 |
| 1686 | GCUUAGCUUGCACCUU GUCACAUAGAGUGAU | ID. NO. 337 |
| 1689 | UAGCUUGCACCUUGUC ACAUAGAGUGAUCUU | ID. NO. 338 |
| 1694 | UGCACCUUGUCACAUA GAGUGAUCUUUCCCA | ID. NO. 339 |
| 1702 | GUCACAUAGAGUGAUC UUUCCCAAGAGAAGG | ID. NO. 340 |
| 1704 | CACAUAGAGUGAUCUU UCCCAAGAGAAGGGG | ID. NO. 341 |
| 1705 | ACAUAGAGUGAUCUUU CCCAAGAGAAGGGGA | ID. NO. 342 |
| 1706 | CAUAGAGUGAUCUUUC CCAAGAGAAGGGGAA | ID. NO. 343 |
| 1727 | AGAAGGGGAAGCACUC GUGUGCAACAGACAA | ID. NO. 344 |
| 1751 | CAGACAAGUGACUGUA UCUGUGUAGACUAUU | ID. NO. 345 |
| 1753 | GACAAGUGACUGUAUC UGUGUAGACUAUUUG | ID. NO. 346 |
| 1759 | UGACUGUAUCUGUGUA GACUAUUUGCUUAUU | ID. NO. 347 |
| 1764 | GUAUCUGUGUAGACUA UUUGCUUAUUUAAUA | ID. NO. 348 |
| 1766 | AUCUGUGUAGACUAUU UGCUUAUUUAAUAAA | ID. NO. 349 |
| 1767 | UCUGUGUAGACUAUUU GCUUAUUUAAUAAAG | ID. NO. 350 |
| 1771 | UGUAGACUAUUUGCUU AUUUAAUAAAGACGA | ID. NO. 351 |
| 1772 | GUAGACUAUUUGCUUA UUUAAUAAAGACGAU | ID. NO. 352 |
| 1774 | AGACUAUUUGCUUAUU UAAUAAAGACGAUUU | ID. NO. 353 |
| 1775 | GACUAUUUGCUUAUUU AAUAAAGACGAUUUG | ID. NO. 354 |
| 1776 | ACUAUUUGCUUAUUUA AUAAAGACGAUUUGU | ID. NO. 355 |
| 1779 | AUUUGCUUAUUUAAUA AAGACGAUUUGUCAG | ID. NO. 356 |
| 1788 | UUUAAUAAAGACGAUU UGUCAGUUGUUUU | ID. NO. 357 |
| 1789 | UUAAUAAAGACGAUUU GUCAGUUGUUUU | ID. NO. 358 |
| 1792 | AUAAAGACGAUUUGUC AGUUGUUUU | ID. NO. 359 |

In addition to all UH sites, Table 3 shows eight potential hairpin ribozyme target sites in stromelysin RNA.

## Table 3

Input Sequence = HUMSTROM.RNA.   Cut Site = RCN GHCB or RCN GUYB.    Stem Length =   15

| Position | Target Sequence | Seq. ID. NO. |
| --- | --- | --- |
| 66 | CUACU GUU GCUGUGCGUGGCAGU | ID. NO. 360 |
| 82 | UGGCA GUU UGCUCAGCCUAUCCA | ID. NO. 361 |
| 192 | AAACA GUU UGUUAGGAGAAAGGA | ID. NO. 362 |

| 430 | AUGCU GUU GAUUCUGCUGUUGAG | ID. NO. 363 |
| 442 | CUGCU GUU GAGAAAGCUCUGAAA | ID. NO. 364 |
| 775 | UCACA GAC CUGACUCGGUUCCGC | ID. NO. 365 |
| 1360 | AUGCU GUU UUUGAAGAAUUUGGG | ID. NO. 366 |
| 1407 | UCACA GUU GGAGUUUGACCCAAA | ID. NO. 367 |

In addition, potential ribozyme target sites within the rabbit stromelysin mRNA sequence (1795 nucleotides) were located and aligned with the human target sites. Because the rabbit stromelysin mRNA sequence has an 84% sequence identity with the human sequence, many ribozyme target sites are also homologous. Thus, the rabbit has potential as an appropriate animal model in which to test ribozymes that are targeted to human stromelysin but have homologous or nearly homologous cleavage sites on rabbit stromelysin mRNA as well. Thirty of the 316 UH sites in the rabbit sequence are identical with the corresponding site in the human sequence with respect to at least 14-nucleotides surrounding the potential ribozyme cleavage sites. The nucleotide in the RNA substrate that is immediately adjacent (5') to the cleavage site is unpaired in the ribozyme-substrate complex (see Fig.1) and is consequently not included in the comparison of human and rabbit potential ribozyme sites. In choosing human ribozyme target sites for continued testing, the presence of identical or nearly identical sites in the rabbit sequence is considered.

Example 2: Superior sites

Potential ribozyme target sites were subjected to further analysis using computer folding programs (Mulfold or a Macintosh-based version of the following program, LRNA (Zucker (1989) Science 244:48), to determine if 1) the target site is substantially single-stranded and therefore predicted to be available for interaction with a ribozyme, 2) if a ribozyme designed to that site is predicted to form stem II but is generally devoid of any other intramolecular base pairing, and 3) if the potential ribozyme and the sequence flanking both sides of the cleavage site together are predicted to interact correctly. The sequence of Stem II can be altered to maintain a stem at that position but minimize intramolecular basepairing with the ribozyme's substrate binding arms. Based on these minimal criteria, and including all the sites that are identical in human and rabbit stromelysin mRNA sequence, a subset of 66 potential superior ribozyme target sites was chosen (as first round targets) for continued analysis. These are SEQ. ID. NOS.: 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359.

Example 3: Accessible sites

To determine if any or all of these potential superior sites might be accessible to a ribozyme directed to that site, an RNAse H assay (described in more detail in McSwiggen, U.S. S. N. 07/883,849 and 07/884,073 both filed May 14, 1992 and hereby incorporated by reference herein). Using this assay, the accessibility of a potential ribozyme target site to a DNA oligonucleotide probe can be assessed without having to synthesize a ribozyme to that particular site. If the complementary DNA oligonucleotide is able to hybridize to the potential ribozyme target site then RNAse H, which has the ability to cleave the RNA of a DNA/RNA hybrid, will be able to cleave the target RNA at that particular site. Specific cleavage of the target RNA by RNAse H is an indication that that site is "open" or "accessible" to oligonucleotide binding and thus predicts that the site will also be open for ribozyme binding. By comparing the relative amount of specific RNAse H cleavage products that are generated for each DNA oligonucleotide/site, potential ribozyme sites can be ranked according to accessibility.

To analyze target sites using the RNAse H assay, DNA oligonucleotides (generally 13-15 nucleotides in length) that are complementary to the potential target sites are synthesized. Body-labeled substrate RNAs (either full-length RNAs or ∼500-600 nucleotide subfragments of the entire RNA) are prepared by *in vitro* transcription in the presence of a $^{32}$P-labeled nucleotide. Unincorporated nucleotides are removed from the $^{32}$P-labeled substrate RNA by spin chromatography on a G-50 Sephadex column and used without further purification. To carry out the assay, the $^{32}$P-labeled substrate RNA is pre-incubated with the specific DNA oligonucleotide (1 µM and 0.1 µM final concentration) in 20 mM Tris-HCl, pH 7.9, 100 mM KCl, 10 mM $MgCl_2$, 0.1 mM EDTA, 0.1 mM DTT at 37°C for 5 minutes. An excess of RNAse H (0.8 units/10 ml reaction) is added and the incubation is continued for 10 minutes. The reaction is quenched by the addition of an equal volume of 95% formamide, 20 mM EDTA, 0.05% bromophenol blue and 0.05% xylene cyanol FF after which the sample is heated to 95°C for 2 minutes, quick chilled and loaded onto a denaturing polyacrylamide gel. RNAse H-cleaved RNA products are separated from uncleaved RNA on denaturing polyacrylamide gels, visualized by autoradiography and the amount of cleavage product is quantified.

RNAse H analysis on the 66 potential ribozyme sites was carried out and those DNA oligonucleotides/sites that supported the most RNAse H cleavage were determined. These assays were carried out using full-length human and rabbit stromelysin RNA as substrates. Results determined on human stromelysin RNA indicated that 23 of the 66 sites supported a high level of RNAse H cleavage, and an additional 13 supported a moderate level of RNAse H cleavage. Twenty-two sites were chosen from among these two groups for continued study. Two of the criteria used for making this choice were 1) that the particular site supported at least moderate RNAse H cleavage on human stromelysin RNA and 2) that the site have two or fewer nucleotide differences between the rabbit and the human stromelysin sequence. RNAse H accessibility on rabbit stromelysin RNA was determined, but was not used as a specific criteria for these choices. Those DNA oligonucleotides that are not totally complementary to the rabbit sequence may not be good indicators of the relative amount of RNAse H cleavage, possibly because the mismatch leads to less efficient hybridization of the DNA oligonucleotide to the mismatched RNA substrate and therefore less RNAse H cleavage is seen.

Example 4: Analysis of Ribozymes

Ribozymes were then synthesized to 22 sites predicted to be accessible as judged the RNAse H assay. Eleven of these 22 sites are identical to the corresponding rabbit sites. The 22 sites are SEQ. ID, NOS.: 34, 35, 57, 125, 126, 127, 128, 129, 140, 162, 170, 179, 188, 223, 224, 236, 245, 246, 256, 259, 260, 281. The 22 ribozymes were chemically synthesized with recognition arms of either 7 nucleotides or 8 nucleotides, depending on which ribozyme alone and ribozyme-substrate combinations were predicted by the computer folding program (Mulfold) to fold most correctly. After synthesis, ribozymes are either purified by HPLC or gel purified.

The sequences of these ribozymes was as follows:

Table 4

| Position | Ribozyme | | | Seq. ID. NO. |
|---|---|---|---|---|
| 10 | GUUGUCUC | CUGAAGAGCACGAAAGUGCGAA | AUGCCUUG | ID. NO. 375 |
| 21 | UUAGCUC | CUGAUGAGGCCGAAAGGCCGAA | AUGUUGU | ID. NO. 376 |
| 168 | GAGGUCG | CUGAUGAGGCCGAAAGGCCGAA | AGUAGUU | ID. NO. 377 |
| 616 | CUCCAUU | CUGAUGAGGCCGAAAGGCCGAA | AUCCCUG | ID. NO. 378 |
| 617 | UCUCCAU | CUGAUGAGGCCGAAAGGCCGAA | AAUCCCU | ID. NO. 379 |
| 633 | CAUCAUCA | CUGAAGAGCACGAAAGUGCGAA | AGUGGGCA | ID. NO. 380 |
| 634 | UCAUCAUC | CUGAAGAGCACGAAAGUGCGAA | AAGUGGGC | ID. NO. 381 |
| 662 | CCUGUUG | CUGAUGAGGCCGAAAGGCCGAA | AUCCUUU | ID. NO. 382 |
| 711 | ACCCAGG | CUGAUGAGGCCGAAAGGCCGAA | AGUGGCC | ID. NO. 383 |
| 820 | GGGACUG | CUGAUGAGGCCGAAAGGCCGAA | AUGCCAU | ID. NO. 384 |
| 883 | UCUGGAGG | CUGAAGAGCACGAAAGUGCGAA | ACAGGUUC | ID. NO. 385 |
| 947 | CCCCUCA | CUGAUGAGGCCGAAAGGCCGAA | AGUGCUG | ID. NO. 386 |
| 996 | CCUGAGG | CUGAUGAGGCCGAAAGGCCGAA | AUUUGCG | ID. NO. 387 |
| 1123 | UGGCCCA | CUGAUGAGGCCGAAAGGCCGAA | AAUUGAU | ID. NO. 388 |
| 1132 | UUUCCUCU | CUGAUGAGCACGAAAGUGCGAA | AUGGCCCA | ID. NO. 389 |
| 1221 | CCUUAUCA | CUGAAGAGCACGAAAGUGCGAA | AAAUGGCU | ID. NO. 390 |
| 1266 | UCUCCAG | CUGAUGAGGCCGAAAGGCCGAA | AUUUGUC | ID. NO. 391 |
| 1275 | UCUCAUCA | CUGAAGAGCACGAAAGUGCGAA | AUCUCCAG | ID. NO. 392 |
| 1334 | AUCCCUG | CUGAUGAGGCCGAAAGGCCGAA | AAAGUCU | ID. NO. 393 |
| 1354 | CAGCAUC | CUGAUGAGGCCGAAAGGCCGAA | AUCUUUG | ID. NO. 394 |
| 1363 | UCUUCAAA | CUGAUGAGCACGAAAGUGCGAA | ACAGCAUC | ID. NO. 395 |
| 1410 | AAACUCC | CUGAUGAGGCCGAAAGGCCGAA | ACUGUGA | ID. NO. 396 |

These 22 ribozymes were then tested for their ability to cleave both human and rabbit full-length stromelysin RNA. Full-length, body-labeled stromelysin RNA is prepared by *in vitro* transcription in the presence of $^{32}$P[CTP], passed over a G 50 Sephadex column by spin chromatography and used as substrate RNA without further purification. Assays are performed by prewarming a 2X concentration of purified ribozyme in ribozyme cleavage buffer (50 mM Tris-HCl, pH 7.5 at 37°C, 10 mM $MgCl_{2)}$ and the cleavage reaction is initiated by adding the 2X ribozyme mix to an equal volume of substrate RNA (maximum of 1-5 nM) that has also been prewarmed in cleavage buffer. As an initial screen, assays are carried out for 1 hour at 37°C using a final concentration of 1 μM and 0.1 μM ribozyme, i.e., ribozyme excess. The reaction is quenched by the addition of an equal volume of 95% formamide, 20 mM EDTA, 0.05% bromophenol blue and 0.05% xylene cyanol FF after which the sample is heated to 95°C for 2 minutes, quick chilled and loaded onto a denaturing polyacrylamide gel. Full-length substrate RNA and the specific RNA products generated by ribozyme cleavage are visualized on an autoradiograph of the gel.

Of the 22 ribozymes tested, 21 were able to cleave human and rabbit substrate RNA *in vitro* in a site-specific manner. In all cases, RNA cleavage products of the appropriate lengths were visualized. The size of the RNA was judged by comparison to molecular weight standards electrophoresed in adjacent lanes of the gel. The fraction of substrate RNA cleaved during a ribozyme reaction can be used as an assessment of the activity of that ribozyme *in vitro.* The activity of these 22 ribozymes on full-length substrate RNA ranged from approximately 10% to greater than 95% of the substrate RNA cleaved in the ribozyme cleavage assay using 1 μM ribozyme as described above. A subset of seven of these ribozymes was chosen for continued study. These seven ribozymes (denoted in Table 5) were among those with the highest activity on both human and rabbit stromelysin RNA. Five of these seven sites have sequence identity between human and rabbit stromelysin RNAs for a minimum of 7 nucleotides in both directions flanking the cleavage site. These sites are 883, 947, 1132, 1221 and 1410. and the ribozymes are SEQ. ID. NOS.: 368, 369, 370, 371, 372, 373, and 374.

Example 5: Arm Length Tests

In order to test the effect of arm length variations on the cleavage activity of a ribozyme to a particular site *in vitro,* ribozymes to these seven sites were designed that had alterations in the binding arm lengths. For each site, a complete set of ribozymes was synthesized that included ribozymes with binding arms of 6 nucleotides, 7 nucleotides, 8 nucleotides, 10 nucleotides and 12 nucleotides, i.e., 5 ribozymes to each site. These ribozymes were gel-purified after synthesis and tested in ribozyme cleavage assays as described above.

After analysis of the 35 ribozymes, five ribozymes with varied arm lengths to each of these seven sites, it was clear that two ribozymes were the most active *in vitro.* These two ribozymes had seven nucleotide arms directed against human sequence cleavage sites of nucleotide 617 and nucleotide 820. These are referred to as RZ 617H 7/7 and RZ 820H 7/7 denoting the human (H) sequence cleavage site (617 or 820) and the arm length on the 5' and 3' side of the ribozyme molecule.

Example 6: Testing the efficacy of ribozymes in cell culture

The two most active ribozymes *in vitro* (RZ 617H 7/7 and RZ 820H 7/7) were then tested for their ability to cleave stromelysin mRNA in the cell. Primary cultures of human or rabbit synovial fibroblasts were used in these experiments. For these efficacy tests, ribozymes with 7 nucleotide arms were synthesized with 2' O methyl modifications on the 5 nucleotides at the 5' end of the molecule and on the 5 nucleotides at the 3' end of the molecule. For comparison, ribozymes to the same sites but with 12 nucleotide arms (RZ 617H 12/12 and RZ 820H 12/12) were also synthesized with the 2' O methyl modifications at the 5 positions at the end of both binding arms. Inactive ribozymes that contain 2 nucleotide changes in the catalytic core region were also prepared for use as controls. The catalytic core in the inactive ribozymes is CUUAUGAGGCCGAAAGGCCGAU versus CUGAUGAGGCCGAAAGGCCGAA in the active ribozymes. The inactive ribozymes show no cleavage activity *in vitro* when measured on full-length RNA in the typical ribozyme cleavage assay at a 1 μM concentration for 1 hour.

The general assay was as follows: Fibroblasts, which produce stromelysin, are serum-starved overnight and ribozymes or controls are offered to the cells the next day. Cells are maintained in serum-free media. The ribozyme can be applied to the cells as free ribozyme, or in association with various delivery vehicles such as cationic lipids (including Transfectam™, Lipofectin™ and Lipofectamine™), conventional liposomes, non-phospholipid liposomes or biodegradable polymers. At the time of ribozyme addition, or up to 3 hours later, Interleukin-1β (typically 20 units/ml) can be added to the cells to induce a large increase in stromelysin expression. The production of stromelysin can then be monitored over a time course, usually up to 24 hours.

If a ribozyme is effective in cleaving stromelysin mRNA within a cell, the amount of stromelysin mRNA will be decreased or eliminated. A decrease in the level of cellular stromelysin mRNA, as well as the appearance of the RNA products generated by ribozyme cleavage of the full-length stromelysin mRNA, can be analyzed by methods such as Northern blot analysis, RNAse protection assays and/or primer extension assays. The effect of ribozyme cleavage of cellular stromelysin mRNA on the production of the stromelysin protein can also be measured by a number of assays. These include the ELISA (Enzyme-Linked Immuno Sorbent Assay) and an immunofluorescence assay described below. In addition, functional assays have been published that monitor stromelysin's enzymatic activity by measuring degradation of its primary substrate, proteoglycan.

Example 7: Analysis of Stromelysin Protein

Stromelysin secreted into the media of Interleukin-1β-induced human synovial fibroblasts was measured by ELISA using an antibody that recognizes human stromelysin. Where present, a Transfectam™-ribozyme complex (0.15 μM ribozyme final concentration) was offered to 2-4 x $10^5$ serum-starved cells for 3 hours prior to induction with Interleukin-1β. The Transfectam™ was prepared according to the manufacturer (Promega Corp.) except that 1:1 (w/w) dioleoyl phosphatidylethanolamine was included. The Transfectam™-ribozyme complex was prepared in a 5:1 charge ratio. Media was harvested 24 hours after the addition of Interleukin-1β. The control (NO RZ) is Transfectam™ alone applied to the cell. Inactive ribozymes, with 7 nucleotide arms or 12 nucleotide arms have the two inactivating changes to the catalytic core that are described above. Cell samples were prepared in duplicate and the assay was carried out on several dilutions of the conditioned media from each sample. Results of the ELISA are presented below as a percent of stromelysin present vs. the control (NO RZ) which is set at 100%.

|  | RZ TARGET SITE | |
| --- | --- | --- |
| TREATMENT | 617H | 820H |
| RZ 7/7 | 6.83 | 7.05 |
| RZ 12/12 | 18.47 | 33.90 |
| INACTIVE RZ 7/7 | 100 | 100 |
| INACTIVE RZ 12/12 | 100 | 100 |
| NO RZ CONTROL | 100 | 100 |

The results above clearly indicate that treatment with active ribozyme, either RZ 617H 7/7 and RZ 820H 7/7, has a dramatic effect on the amount of stromelysin secreted by the cells. When compared to untreated, control cells or cells treated with inactive ribozymes, the level of stromelysin was decreased by approximately 93%. Ribozymes to the same sites, but synthesized with 12 nucleotide binding arms, were also efficacious, causing a decrease in stromelysin to ~66 to ~81% of the control. In previous *in vitro* ribozyme cleavage assays, RZ 617H 7/7 and RZ 820H 7/7 had better cleavage activity on full-length RNA substrates than ribozymes with 12 nucleotide arms directed to the same sites (617H 12/12 and RZ 820H 12/12).

Example 8: Immunofluorescent Assay

An alternative method of stromelysin detection is to visualize stromelysin protein in the cells by immunofluorescence. For this assay, cells are treated with monensin to prevent protein secretion from the cell. The stromelysin retained by the cells after monensin addition can then be visualized by immunofluorescence using either conventional or confocal microscopy. Generally, cells were serum-starved overnight and treated with ribozyme the following day for several hours. Monensin was then added and after ~5-6 hours, monensin-treated cells were fixed and permeabilized by standard methods and incubated with an antibody recognizing human stromelysin. Following an additional incubation period with a secondary antibody that is conjugated to a fluorophore, the cells were observed by microscopy. A decrease in the amount of fluorescence in ribozyme-treated cells, compared to cells treated with inactive ribozymes or media alone, indicates that the level of stromelysin protein has been decreased due to ribozyme treatment.

As visualized by the immunofluorescence technique described above, treatment of human synovial fibroblasts with either RZ 617H 7/7 or RZ 820H 7/7 (final concentrations of 1.5 µM free ribozyme or 0.15 µM ribozyme complexed with Transfectam™ resulted in a significant decrease in fluorescence, and therefore stromelysin protein, when compared with controls. Controls consisted of treating with media or Transfectam™ alone. Treatment of the cells with the corresponding inactive ribozymes with two inactivating changes in the catalytic core resulted in immunofluorescence similar to the controls without ribozyme treatment.

Rabbit synovial fibroblasts were also treated with RZ 617H 7/7 or RZ 820H 7/7, as well as with the two corresponding ribozymes (RZ 617R 7/7 or RZ 820R 7/7) that each have the appropriate one nucleotide change to make them completely complementary to the rabbit target sequence. Relative to controls that had no ribozyme treatment, immunofluorescence in Interleukin-1β-induced rabbit synovial fibroblasts was visibly decreased by treatment with these four ribozymes, whether specific for rabbit or human mRNA sequence. For the immunofluorescence study in rabbit synovial fibroblasts, the antibody to human stromelysin was used.

Example 9: Ribozyme Cleavage of Cellular RNA

The following method was used in this example.

Primer extension assay:

The primer extension assay was used to detect full-length RNA as well as the 3' ribozyme cleavage products of the RNA of interest. The method involves synthesizing a DNA primer (generally ~20 nucleotides in length) that can hybridize to a position on the RNA that is downstream (3') of the putative ribozyme cleavage site. Before use, the primer was labeled at the 5' end with $^{32}$P[ATP] using T4 polynucleotide kinase and purified from a gel. The labeled primer was then incubated with a population of nucleic acid isolated from a cellular lysate by standard procedures. The reaction buffer was 50 mM Tris-HCl, pH 8.3, 3 mM $MgCl_2$, 20 mM KCl, and 10 mM DTT. A 30 minute extension reaction follows, in which all DNA primers that have hybridized to the RNA were substrates for reverse transcriptase, an enzyme that will add nucleotides to the 3' end of the DNA primer using the RNA as a template. Reverse transcriptase was obtained from Life Technologies and is used essentially as suggested by the manufacturer. Optimally, reverse transcriptase will extend the DNA primer, forming cDNA, until the end of the RNA substrate is reached. Thus, for ribozyme-cleaved RNA substrates, the cDNA product will be shorter than the resulting cDNA product of a full-length, or uncleaved RNA substrate. The differences in size of the $^{32}$P-labeled cDNAs produced by extension can then be discriminated by electrophoresis on a denaturing polyacrylamide gel and visualized by autoradiography.

Strong secondary structure in the RNA substrate can, however, lead to premature stops by reverse transcriptase. This background of shorter cDNAs is generally not a problem unless one of these prematurely terminated products electrophoreses in the expected position of the ribozyme-cleavage product of interest. Thus, 3' cleavage products are easily identified based on their expected size and their absence from control lanes. Strong stops due to secondary structure in the RNA do, however, cause problems in trying to quantify the total full-length and cleaved RNA present. For this reason, only the relative amount of cleavage can easily be determined.

The primer extension assay was carried out on RNA isolated from cells that had been treated with Transfectam™-complexed RZ 617H 7/7, RZ 820H 7/7, RZ 617H 12/12 and RZ 820H 12/12. Control cells had been treated with Transfectam™ alone. Primer extensions on RNA from cells treated with the Transfectam™ complexes of the inactive versions of these four ribozymes were also prepared. The 20 nucleotide primer sequence is 5' AATGAAAACGAGGTCCTTGC 3' and it is complementary to a region about 285 nucleotides downstream of ribozyme site 820. For ribozymes to site 617, the cDNA length for the 3' cleavage product is 488 nucleotides, for 820 the cDNA product is 285 nucleotides. Full-length cDNA will be 1105 nucleotides in length. Where present, 1 ml of 0.15 µM ribozyme was offered to ~2-3 x $10^5$ serum-starved human synovial fibroblasts. After 3 hours, 20 units/ml Interleukin-1β was added to the cells and the incubation continued for 24 hours.

$^{32}$P-labeled cDNAs of the correct sizes for the 3' products were clearly visible in lanes that contained RNA from cells that had been treated with active ribozymes to sites 617 and 820. Ribozymes with 7 nucleotide arms were judged to be more active than ribozymes with 12 nucleotide arms by comparison of the relative amount of 3' cleavage product visible. This correlates well with the data obtained by ELISA analysis of the conditioned media from these same samples. In addition, no cDNAs corresponding to the 3' cleavage products were visible following treatment of the cells with any of the inactive ribozymes.

To insure that ribozyme cleavage of the RNA substrate was not occurring during the preparation of the cellular RNA or during the primer extension reaction itself, several controls have been carried out. One control was to add body-labeled stromelysin RNA, prepared by *in vitro* transcription, to the cellular lysate. This lysate was then subjected to the typical RNA preparation and primer extension analysis except that non-radioactive primer was used. If ribozymes that are present in the cell at the time of cell lysis are active under any of the conditions during the subsequent analysis, the added, body-labeled stromelysin RNA will become cleaved. This, however, is not the case. Only full-length RNA was visible by gel analysis, no ribozyme cleavage products were present. This is evidence that the cleavage products detected in RNA from ribozyme-treated cells resulted from ribozyme cleavage in the cell, and not during the subsequent analysis.

Example 10: RNAse Protection Assay

By RNAse protection analysis, both the 3' and the 5' products generated by ribozyme cleavage of the substrate RNA in a cell can be identified. The RNAse protection assay is carried out essentially as described in the protocol provided with the Lysate Ribonuclease Protection Kit (United States Biochemical Corp.) The probe for RNAse protection is an RNA that is complementary to the sequence surrounding the ribozyme cleavage site. This "antisense" probe RNA is transcribed *in vitro* from a template prepared by the polymerase chain reaction in which the 5' primer was a DNA oligonucleotide containing the T7 promoter sequence. The probe RNA is body labeled during transcription by including $^{32}$P[CTP] in the reaction and purified away from unincorporated nucleotide triphosphates by chromatography on G-50 Sephadex. The probe RNA (100,000 to 250,000 cpms) is allowed to hybridize overnight at 37°C to the RNA from a cellular lysate or to RNA purified from a cell lysate. After hybridization, RNAse $T_1$ and RNAse A are added to degrade all single-stranded RNA and the resulting products are analyzed by gel electrophoresis and autoradiography. By this analysis, full-length, uncleaved target RNA will protect the full-length probe. For ribozyme-cleaved target RNAs, only a portion of the probe will be protected from RNAse digestion because the cleavage event has occurred in the region to which the probe binds. This results in two protected probe fragments whose size reflects the position at which ribozyme cleavage occurs and whose sizes add up to the size of the full-length protected probe.

RNAse protection analysis was carried out on cellular RNA isolated from rabbit synovial fibroblasts that had been treated either with active or inactive ribozyme. The ribozymes tested had 7 nucleotide arms specific to the rabbit sequence but corresponding to human ribozyme sites 617 and 820 (i.e. RZ 617R 7/7, RZ 820R 7/7). The inactive ribozymes to the same sites also had 7 nucleotide arms and included the two inactivating changes described above. The inactive ribozymes were not active on full-length rabbit stromelysin RNA in a typical 1 hour ribozyme cleavage reaction *in vitro* at a concentration of 1 µM. For all samples, one ml of 0.15 µM ribozyme was administered as a Transfectam™ complex to serum-starved cells. Addition of Interleukin-1β followed 3 hours later and cells were harvested after 24 hours. For samples from cells treated with either active ribozyme tested, the appropriately-sized probe fragments representing ribozyme cleavage products were visible. For site 617, two fragments corresponding to 125 and 297 nucleotides were present, for site 820 the two fragments were 328 and 94 nucleotides in length. No protected probe fragments representing RNA cleavage products were visible in RNA samples from cells that not been treated with any ribozyme, or in cells that had received the inactive ribozymes. Full-length protected probe (422 nucleotides in length) was however visible, indicating the presence of full-length, uncleaved stromelysin RNA in these samples.

Delivery of Free and Transfectam-Complexed Ribozymes to Fibroblasts

Ribozymes can be delivered to fibroblasts complexed to a cationic lipid or in free form. To deliver free ribozyme, an appropriate dilution of stock ribozyme (final concentration is usually 1.5 µM) is made in serum-free medium; if a radioactive tracer is to be used (i.e., $^{32}$P), the specific activity of the ribozyme is adjusted to 800-1200 cpm/pmol. To deliver ribozyme complexed with the cationic lipid Transfectam, the lipid is first prepared as a stock solution containing 1/1 (w/w) dioleoylphosphatidylcholine (DOPE). Ribozyme is mixed with the Transfectam/DOPE mixture at a 1/5 (RZ/TF) charge ratio; for a 36-mer ribozyme, this is a 45-fold molar excess of Transfectam (Transfectam has 4 positive charges per molecule). After a 10 min incubation at room temperature, the mixture is diluted and applied to cells, generally at a ribozyme concentration of 0.15 µM. For $^{32}$P experiments, the specific activity of the ribozyme is the same as for the free ribozyme experiments.

After 24 hour, about 30% of the offered Transfectamribozyme cpm's are cell-associated (in a nuclease-resistant manner). Of this, about 10-15% of the cpm's represent intact ribozyme; this is about 20-25 million ribozymes per cell. For the free ribozyme, about 0.6% of the offered dose is cell-associated after 24 hours. Of this, about 10-15% is intact; this is about 0.6-0.8 million ribozymes per cell.

Administration of Ribozyme

Selected ribozymes can be administered prophylactically, or to patients having arthritic conditions, e.g., by exogenous delivery of the ribozyme to a desired tissue by means of an appropriate delivery vehicle, e.g., cationic lipids, non-phospholipid liposomes, biodegradable polymers, a liposome, a controlled release vehicle, by use of iontophoresis, electroporation or ion paired molecules, or covalently attached adducts, and other pharmacologically approved methods of delivery. Routes of administration include intra-articular, intramuscular, aerosol, oral (tablet or pill form), topical, systemic, ocular, intraperitoneal and/or intrathecal. Expression vectors for immunization with ribozymes and/or delivery of ribozymes are also suitable. Such ribozymes are also useful in treatment of cancer and angiogenesis.

The specific delivery route of any selected ribozyme will depend on the use of the ribozyme. Generally, a specific delivery program for each ribozyme will focus on unmodified ribozyme uptake with regard to intracellular localization, followed by demonstration of efficacy. Alternatively, delivery to these same cells in an organ or tissue of an animal can be pursued. Uptake studies will include uptake assays to evaluate cellular ribozyme uptake, regardless of the delivery vehicle or strategy. Such assays will also determine the intracellular localization of the ribozyme following uptake, ultimately establishing the requirements for maintenance of seady-state concentrations within the cellular compartment containing the target sequence (nucleus and/or cytoplasm). Efficacy and cytotoxicity can then be tested. Toxicity will not only include cell viability but also cell function.

Some methods of delivery that may be used include:

    a. encapsulation in liposomes,
    b. transduction by retroviral vectors,
    c. conjugation with cholesterol,
    d. localization to nuclear compartment utilizing nuclear targeting site found on most nuclear proteins,
    e. neutralization of charge of ribozyme by using nucleotide derivatives,
    f. use of blood stem cells to distribute ribozymes throughout the body, and
    g. tranfection ex vivo and in vivo.

At least three types of delivery strategies are useful in the present invention, including: ribozyme modifications, particle carrier drug delivery vehicles, and retroviral expression vectors. Unmodified ribozymes, like most small molecules, are taken up by cells, albeit slowly. To enhance cellular uptake, the ribozyme may be modified essentially at random, in ways which reduce its charge but maintains specific functional groups. This results in a molecule which is able to diffuse across the cell membrane, thus removing the permeability barrier.

Modification of ribozymes to reduce charge is just one approach to enhance the cellular uptake of these larger molecules. The random approach, however, is not advisable since ribozymes are structurally and functionally more complex than small drug molecules. The structural requirements necessary to maintain ribozyme catalytic activity are well understood by those in the art. These requirements are taken into consideration when designing modifications to enhance cellular delivery. The modifications are also designed to reduce susceptibility to nuclease degradation. Both of these characteristics should greatly improve the efficacy of the ribozyme. Cellular uptake can be increased by several orders of magnitude without having to alter the phosphodiester linkages necessary for ribozyme cleavage activity.

Chemical modifications of the phosphate backbone will reduce the negative charge allowing free diffusion across the membrane. This principle has been successfully demonstrated for antisense DNA technology. The similarities in chemical composition between DNA and RNA make this a feasible approach. In the body, maintenance of an external concentration will be necessary to drive the diffusion of the modified ribozyme into the cells of the tissue. Administration routes which allow the diseased tissue to be exposed to a transient high concentration of the drug, which is slowly dissipated by systemic adsorption are preferred. Intravenous administration with a drug carrier designed to increase the circulation half-life of the ribozyme can be used. The size and composition of the drug carrier restricts rapid clearance from the blood stream. The carrier, made to accumulate at the site of infection, can protect the ribozyme from degradative processes.

Drug delivery vehicles are effective for both systemic and topical administration. They can be designed to serve as a slow release reservoir, or to deliver their contents directly to the target cell. An advantage of using direct delivery drug vehicles is that multiple molecules are delivered per uptake. Such vehicles have been shown to increase the circulation half-life of drugs which would otherwise be rapidly cleared from the blood stream. Some examples of such specialized drug delivery vehicles which fall into this category are liposomes, hydrogels, cyclodextrins, biodegradable nanocapsules, and bioadhesive microspheres.

From this category of delivery systems, liposomes are preferred. Liposomes increase intracellular stability, increase uptake efficiency and improve biological activity.

Liposomes are hollow spherical vesicles composed of lipids arranged in a similar fashion as those lipids which make up the cell membrane. They have an internal aqueous space for entrapping water soluble compounds and range in size from 0.05 to several microns in diameter. Several studies have shown that liposomes can deliver RNA to cells and that the RNA remains biologically active.

For example, a liposome delivery vehicle originally designed as a research tool, Lipofectin, has been shown to deliver intact mRNA molecules to cells yielding production of the corresponding protein.

Liposomes offer several advantages: They are nontoxic and biodegradable in composition; they display long circulation half-lives; and recognition molecules can be readily attached to their surface for targeting to tissues. Finally, cost effective manufacture of liposome-based pharmaceuticals, either in a liquid suspension or lyophilized product, has demonstrated the viability of this technology as an acceptable drug delivery system.

Other controlled release drug delivery systems, such as nonoparticles and hydrogels may be potential delivery vehicles for a ribozyme. These carriers have been developed for chemotherapeutic agents and protein-based pharmaceuticals, and consequently, can be adapted for ribozyme delivery.

Topical administration of ribozymes is advantageous since it allows localized concentration at the site of administration with minimal systemic adsorption. This simplifies the delivery strategy of the ribozyme to the disease site and reduces the extent of toxicological characterization. Furthermore, the amount of material to be applied is far less than that required for other administration routes. Effective delivery requires the ribozyme to diffuse into the infected cells. Chemical modification of the ribozyme to neutralize negative charge may be all that is required for penetration. However, in the event that charge neutralization is insufficient, the modified ribozyme can be co-formulated with permeability enhancers, such as Azone or oleic acid, in a liposome. The liposomes can either represent a slow release presentation vehicle in which the modified ribozyme and permeability enhancer transfer from the liposome into the infected cell, or the liposome phospholipids can participate directly with the modified ribozyme and permeability enhancer in facilitating cellular delivery. In some cases, both the ribozyme and permeability enhancer can be formulated into a suppository formulation for slow release.

Ribozymes may also be systemically administered. Systemic absorption refers to the accumulation of drugs in the blood stream followed by distribution throughout the entire body. Administration routes which lead to systemic absorption include: intravenous, subcutaneous, intraperitoneal, intranasal, intrathecal and ophthalmic. Each of these administration routes expose the ribozyme to an accessible diseased tissue. Subcutaneous administration drains into a localized lymph node which proceeds through the lymphatic network into the circulation. The rate of entry into the circulation has been shown to be a function of molecular weight or size. The use of a liposome or other drug carrier localizes the ribozyme at the lymph node. The ribozyme can be modified to diffuse into the cell, or the liposome can directly participate in the delivery of either the unmodified or modified ribozyme to the cell.

A liposome formulation which can deliver oligonucleotides to lymphocytes and macrophages is also useful for certain conditions. This oligonucleotide delivery system prevents mRNA expression in affected primary immune cells. Whole blood studies show that the formulation is taken up by 90% of the lymphocytes after 8 hours at 37°C.

Preliminary biodistribution and pharmacokinetic studies yielded 70% of the injected dose/gm of tissue in the spleen after one hour following intravenous administration.

Liposomes injected intravenously show accumulation in the liver, lung and spleen. The composition and size can be adjusted so that this accumulation represents 30% to 40% of the injected dose. The remaining dose circulates in the blood stream for up to 24 hours.

The chosen method of delivery should result in cytoplasmic accumulation and molecules should have some nuclease-resistance for optimal dosing. Nuclear delivery may be used but is less preferable. Most preferred delivery methods include liposomes (10-400 nm), hydrogels, controlled-release polymers, microinjection or electroporation (for *ex vivo* treatments) and other pharmaceutically applicable vehicles. The dosage will depend upon the disease indication and the route of administration but should be between 100-200 mg/kg of body weight/day. The duration of treatment will extend through the course of the disease symptoms, possibly continuously. The number of doses will depend upon disease delivery vehicle and efficacy data from clinical trials.

Establishment of therapeutic levels of ribozyme within the cell is dependent upon the rate of uptake and degradation. Decreasing the degree of degradation will prolong the intracellular half-life of the ribozyme. Thus, chemically modified ribozymes, <u>e.g.</u>, with modification of the phosphate backbone, or capping of the 5' and 3' ends of the ribozyme with nucleotide analogs may require different dosaging. Descriptions of useful systems are provided in the art cited above.

Other embodiments are within the following claims.

**Claims**

1. Use of an enzymatic RNA molecule which cleaves target mRNA having a sequence selected from SEQ. ID. NOS. 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210; 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, and 353, in the manufacture of a medicament for the treatment of arthritis.

2. The use of an enzymatic RNA molecule of claim 1, which cleaves target mRNA having a sequence selected from SEQ. ID. NOS. 34, 35, 57, 125, 126, 127, 128, 129, 140, 162, 10 170, 179, 188, 223, 224, 236, 245, 246, 256, 259, 260, and 281.

3. An enzymatic RNA molecule suitable for treating arthritis, which cleaves target RNA having a sequence selected from SEQ. ID. NOS. 368, 369m, 371, 372 and 374.

4. The use of claims 1 or 2 or the enzymatic RNA molecule of claim 3 wherein said RNA molecule is in a hammerhead motif.

5. The use of claim 1 wherein said enzymatic RNA molecule is in a hairpin, hepatitis Delta virus, group 1 intron or RNAseP RNA motif.

6. The use of claim 1 wherein said enzymatic RNA molecule comprises between 5 and 23 bases complementary to said mRNA.

7. The enzymatic RNA molecule of claim 3 wherein said RNA molecule comprises between 10 and 18 bases complementary to said mRNA.

**8.** The enzymatic RNA molecule of claim 3 wherein said RNA molecule comprises between 30 and 45 bases complementary to said mRNA.

**9.** A mammalian cell including an enzymatic RNA molecule of

**10.** The cell of claim 9 wherein said cell is a human cell.

**11.** An expression vector including nucleic acid encoding the enzymatic RNA molecule of claim 3 in a manner which allows expression of that enzymatic RNA molecule within a mammalian cell.

**12.** Use of a molecule of any of claims 1, 2 or 3 in the production of a pharmaceutical composition for the treatment of arthritis.

**Patentansprüche**

**1.** Verwendung eines enzymatischen RNA Moleküls, welches Ziel-mRNA mit einer Sequenz spaltet, die ausgewählt ist aus den SEQ. ID. Nrn. 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 128, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352 und 353 zur Herstellung eines Medikamentes für die Behandlung von Arthritis.

**2.** Die Verwendung eines enzymatischen RNA Moleküls nach Anspruch 1, welches Ziel-mRNA mit einer Sequenz spaltet, die ausgewählt ist aus den SEQ. ID. Nrn. 34, 35, 57, 125, 126, 127, 128, 129, 140, 162, 10 170, 179, 188, 223, 224, 236, 245, 246, 256, 259, 260 und 281.

**3.** Ein zum Behandeln von Arthritis geeignetes enzymatisches RNA Molekül, welches Ziel-RNA mit einer Sequenz spaltet, die ausgewählt ist aus den SEQ. ID. Nrn. 368, 369m, 371, 372 und 374.

**4.** Die Verwendung nach den Ansprüchen 1 oder 2 oder des enzymatischen RNA Moleküls nach Anspruch 3, wobei das RNA Molekül in einem Hammerkopf-Motiv vorliegt.

**5.** Die Verwendung nach Anspruch 1, wobei das enzymatische RNA Molekül in einem Haarnadel-, Hepatitis Delta Virus -, Gruppe 1 Intron - oder RNAseP RNA -Motiv vorliegt.

**6.** Die Verwendung nach Anspruch 1, wobei das enzymatische RNA Molekül zwischen 5 und 23 zu der mRNA komplementäre Basen umfasst.

**7.** Das enzymatische RNA Molekül nach Anspruch 3, wobei das RNA Molekül zwischen 10 und 18 zu der mRNA komplementäre Basen umfasst.

**8.** Das enzymatische RNA Molekül nach Anspruch 3, wobei das RNA Molekül zwischen 30 und 45 zu der mRNA komplementäre Basen umfasst.

**9.** Eine ein enzymatisches RNA Molekül nach Anspruch 3 einschließende Säugerzelle.

**10.** Die Zelle nach Anspruch 9, wobei die Zelle eine menschliche Zelle ist.

**11.** Ein Expressionsvektor, der Nukleinsäure, die für das enzymatische RNA Molekül nach Anspruch 3 kodiert, in einer Weise einschließt, welche die Expression dieses enzymatischen RNA Moleküls in einer Säugerzelle erlaubt.

**12.** Verwendung eines Moleküls nach einem der Ansprüche 1, 2 oder 3 zur Produktion einer pharmazeutischen Zusammensetzung für die Behandlung von Arthritis.

**Revendications**

**1.** Utilisation d'une molécule d'ARN enzymatique qui coupe des ARNm cibles ayant une séquence choisie parmi les SEQ.ID. NOS. 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, et 353, pour la fabrication d'un médicament pour le traitement de l' arthrite.

**2.** Utilisation d'une molécule d'ARN enzymatique selon la revendication 1 qui coupe des ARNm cibles ayant une séquence choisie parmi les SEQ.ID.NOS 34, 35, 57, 125, 126, 127, 128, 129, 140, 162, 170, 179, 188, 223, 224, 236, 245, 246, 256, 259, 260, et 281.

**3.** Une molécule d'ARN enzymatique adaptée au traitement de l'arthrite qui coupe des ARN cibles ayant une séquence choisie parmi les SEQ.ID.NOS. 368, 369m, 371, 372 et 374.

**4.** L'utilisation selon les revendications 1 ou 2 d'une molécule d'ARN enzymatique selon la revendication 3 dans laquelle ladite molécule d'ARN se présente sous la forme d'un motif en tête de marteau.

**5.** L'utilisation selon la revendication 1 dans laquelle ladite molécule d'ARN est en épingle à cheveux, un virus delta de l'hépatite, un intron de groupe 1 ou un motif ARN de l'ARNseP.

**6.** L'utilisation selon la revendication 1 dans laquelle ladite molécule d'ARN comprend entre 5 et 23 bases complémentaires du dit ARNm.

**7.** Une molécule d'ARN enzymatique selon la revendication 3 dans laquelle ladite molécule d'ARN comprend entre 10 et 18 bases complémentaires du dit ARNm.

**8.** Une molécule d'ARN enzymatique selon la revendication 3 dans laquelle ladite molécule d'ARN comprend entre 30 et 45 bases complémentaires du dit ARNm.

**9.** Une cellule de mammifère comprenant une molécule d'ARN enzymatique selon la revendication 3.

**10.** La cellule selon la revendication 9 dans laquelle ladite cellule est une cellule humaine.

**11.** Une vecteur d'expression comprenant un acide nucléique codant pour une molécule d'ARN enzymatique selon la revendication 3 de telle manière qu'elle permette l'expression de cette molécule d'ARN enzymatique dans une cellule de mammifère.

**12.** Utilisation d'une molécule selon l'une quelconque des revendications 1, 2 ou 3 pour 1a préparation d'une composition pharmaceutique pour le traitement de l'arthrite.

# Figure 1: Hammerhead Ribozyme

- Size: ~30 to 40 nucleotides
- Requires the target sequence: UH (C, A or U)
- Binds a variable number of nucleotides on both sides of the cleavage site